# EUROPEAN PATENT SPECIFICATION

(11) **EP 0 543 195 B1**
(45) Date of publication and mention of the grant of the patent: **28.01.1998**
(21) Application number: 92118598.9
(22) Date of filing: 30.10.1992
(51) Int. Cl.: C12N 15/52, C12N 15/62, C12P 21/02, C07K 1/113, C07K 14/31, C12N 15/31

(54) **Biosynthetic process for the preparation of proteins**
Biosynthetisches Verfahren zur Herstellung von Protein
Procédé biosynthétique pour la préparation de protéines

(30) Priority: 31.10.1991 US 784234
(43) Date of publication of application: 26.05.1993
(73) Proprietor: Dr. Karl Thomae GmbH, D-88397 Biberach (DE)
(72) Inventor: Entian, Karl-Dieter, Dr., W-6370 Oberursel/Ts. 5 (DE); Götz, Friedrich, Dr., W-7400 Tübingen (DE); Schnell, Norbert, Dr., W-8630 Coburg (DE); Augustin, Johannes, Dr., W-7400 Tübingen (DE); Engelke, Germar, Dr., W-6000 Frankfurt/Main (DE); Rosenstein, Ralf, Dr., W-7410 Reutlingen 22 (DE); Kaletta, Cortina, Dr., W-8000 München 70 (DE); Klein, Cora, Dr., W-6050 Offenbach (DE); Wieland, Bernd, Dr., W-7407 Rottenburg (DE); Kupke, Thomas, Dr., W-7400 Tübingen (DE); Jung, Günther, Dr., W-7400 Tübingen (DE); Kellner, Roland, Dr., W-6148 Heppenheim (DE)
(74) Representative: Laudien, Dieter, Dr.

(56) References cited:
- EP-A- 0 342 658
- NATURE vol. 333, no. 6170 , 19 May 1988 , LONDON GB pages 276 - 278 NORBERT SCHNELL ET AL. 'Prepeptide sequence of epidermin, a ribosomally synthesized antibiotic with four sulphide-rings'
- JOURNAL OF BACTERIOLOGY vol. 174, no. 16 , August 1992 pages 5354 - 5361 THOMAS KUPKE ET AL. 'Purification and characterization of EpiD, a flavoprotein involved in the biosynthesis of the lantibiotic Epidermin'
- EUROPEAN JOURNAL OF BIOCHEMISTRY vol. 204, no. 1 , February 1992 pages 57 - 68 N. SCHNELL ET AL. 'Analysis of genes involved in the biosynthesis of lantibiotic epidermin'
- EUROPEAN JOURNAL OF BIOCHEMISTRY vol. 204, no. 3 , March 1992 pages 1149 - 1154 JOHANNES AUGUSTIN ET AL. 'Genetic analysis of epidermin biosynthetic genes and epidermin-negative mutants of Staphylococcus epidermidis'
- PROCEEDINGS OF THE 1ST INTERNATIONAL WORKSHOP ON LANTIBIOTICS. NISIN NOVEL LANTIBIOTICS 1991 pages 277 - 286 JOHANNES AUGUSTIN ET AL. 'Identification of epidermin biosynthetic genes by complementation studies and heterologous expression'
- PROCEEDINGS OF THE 1ST INTERNATIONAL WORKSHOP ON LANTIBIOTICS. NISIN NOVEL LANTIBIOTICS 1991 pages 269 - 276 NORBERT SCHNELL ET AL. 'The operon-like organisation of lamtibiotic epidermin biosynthesis genes'

## Description

### Field of the Invention

This invention relates to the biosynthesis of chemical compounds, and in particular to the biosynthesis of chemical compounds containing dehydroamino acid residues and/or thioether bridges. The invention also relates to the use of recombinant genetics to prepare enzymes involved in the biosynthesis of such chemical compounds.

### Background of the Invention

Some polypeptide antibodies such as nisin, subtilin, duramycin, cinnamycin, ancovenin, Ro 09-0198 and epidermin contain dehydroamino acids and lanthionine bridges. These polypeptides are produced by various respective strains of microorganism. Nisin for example can be produced by cultivating strains of *Streptococcus lactis*, and subtilin by cultivation of *Bacillus subtilis.*

The genetic basis for the biosynthesis of these antibiotics has not, hitherto, been elucidated. It was only known from Schnell et al. (Nature 1988, 333, pp 276 - 278), that the structural gene epiA from Staphylococcus epidermidis (DSM 3095) encodes epidermin. Epi A is located on a 54kb plasmid and encodes for a 52 amino acid propeptide. Whether biosynthesis of such antibiotics and, in particular, the formation of the unusual amino acids found therein occurs via ribosomal synthesis or via multi-enzyme complex, has not been known.

In addition it was not known whether the precursor proteins of such antibiotics were coded by distinct structural genes or were the degradation products of larger proteins.

In the course of work carried out to establish the structural gene of epiderm, we have been able to establish that surprisingly the above mentioned antibiotics, in particular epidermin, are each coded by a distinct structural gene, and that processing of a presequence polypeptide is carried out by an enzymatic complex which effects formation of dehydroamino residues and/or thioether bridges.

Furthermore, the multi-enzyme complex may be involved in the secretion of the protein through the cell membrane into the culture supernatant, as well as processing a prepolypeptide. In this connection, such activity may be associated with a pre-sequence possessed by the pre-polypeptide, e.g., as in the case of the -30 to -1 sequence of pre-epidermin as described below.

### Description of the Drawings

Figure 1 depicts the nucleotide sequence of the epidermin structural gene (epi A) and the deduced amino acid sequence of pre-epidermin. A Shine-Dalgarno sequence is boxed and the proteolytic cleavage site at which the propeptide is processed is indicated by an arrow. Inverted repeats are underlined and potential stop codons are noted as am (amber) and oc (ochre).

Figure 2A depicts a prediction plot for pre-epidermin using a Hyron program, in which the respective bar charts show: (a) flexibility; (b) hydropathy; (c) hydrophilicity; (d) propensities for turn; (e) β-sheet; and (f) α-helix conformation.

Figure 2B depicts a helix wheel plot for pre-epidermin showing that the N-terminus may partially adopt an amphophilic α-helical conformation in an appropriate environment.

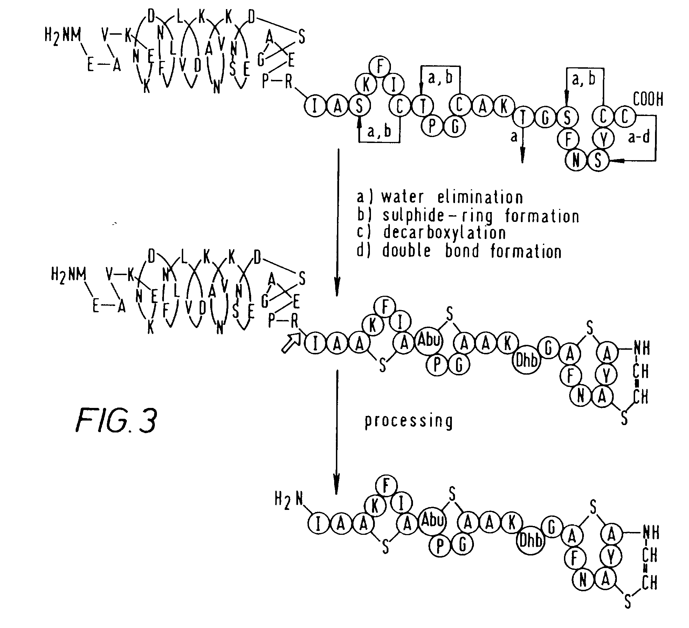

Figure 3 depicts a postulated maturation procedure for epidermin. The translated polypeptide (pre-epidermin) consists of 52-amino acid residues. Structure predictions indicate a partially α-helical N-terminus from which residues -30 to -10 may form an amphilphilic α-helix conformation. Water elimination occurs at the indicated Ser and Thr residues (a). With the exception of Thr⁺¹⁴, water elimination is followed by sulphide ring formation (b) and at the C-terminus, decarboxylation (c) and double bond formation (d) to produce pro-epidermin. The pro-epidermin structure is then processed by proteolytic cleavage to produce epidermin.

Figure 4 depicts the structure of epidermin. The ring structures are designated as A, B, C, D and E. The structures of the amino acids mesolanthione arid threo-methyllanthione, are set forth.

Figure 5 depicts examples of unusual amino acids which are found in lanthione antibiotics and which can be formed in peptide products using the method of this invention.

Figure 6 depicts a schematic representation of the process for preparation of the pCUI plasmid from pCLP100 plasmid and pUC18 plasmid.

Figure 7 depicts the elution pattern of the isolated culture medium prepared in Example 2.

Figure 7B depicts the elution pattern of a standard containing gallidermin. Gallidermin is eluted at 7.54 minutes.

Figure 8 is a genetic analysis of episome pTü32 of *S. epidermidis* plasmid pTü32, including
- 8A:: a restriction map of episome pTü32, and
- 8B:: a restriction map of the 13.5 kb BglII fragment of pTü32. The filled arrow corresponds to the epiA structural gene. Open arrows represent reading frames epiB, C, D, P and Q.
- 8C:: Southern hybridization of pTü32 digested with different restriction enzymes (EcoRI, EcoRV, BglIII, SphI) using a 15-mer oligonucleotide (5'CACATCCAGGAGTAC-3') specific of epiA.

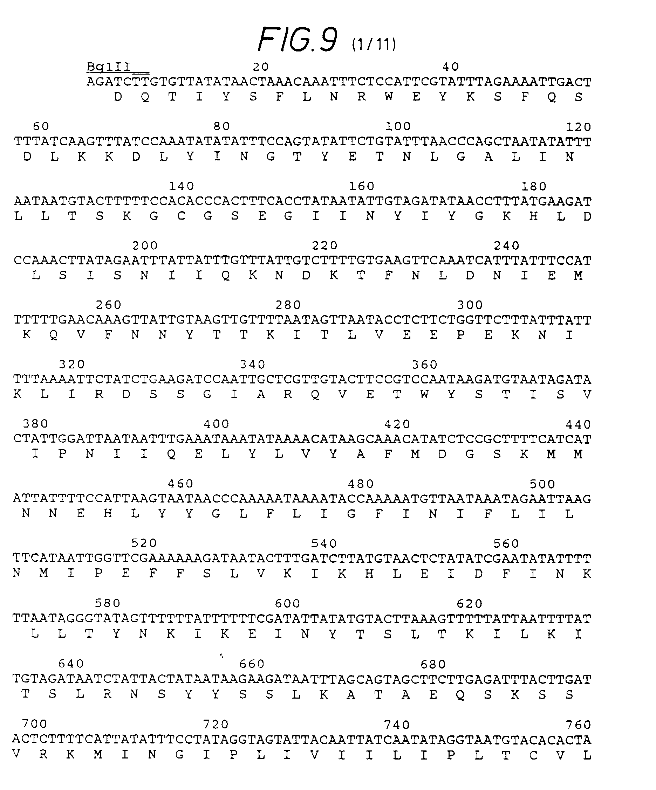

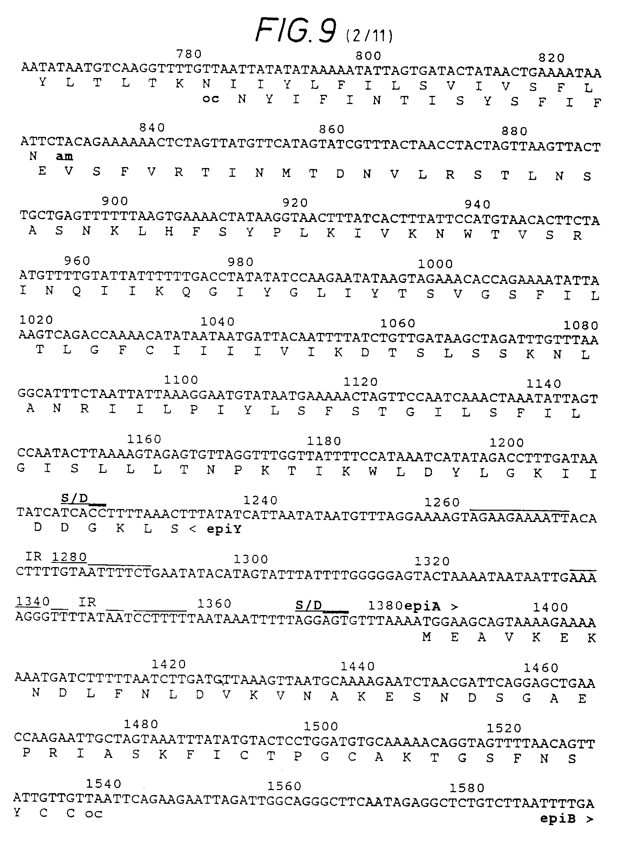

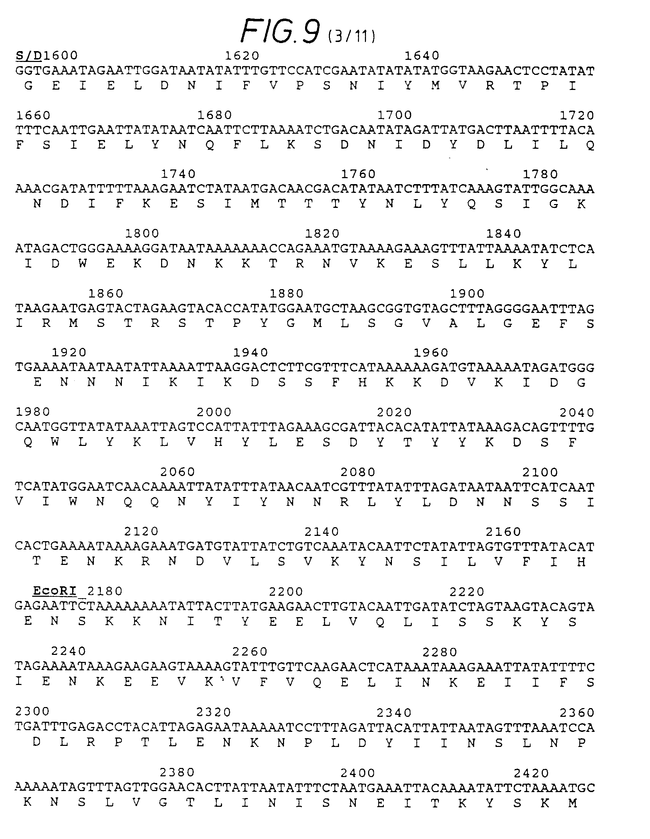

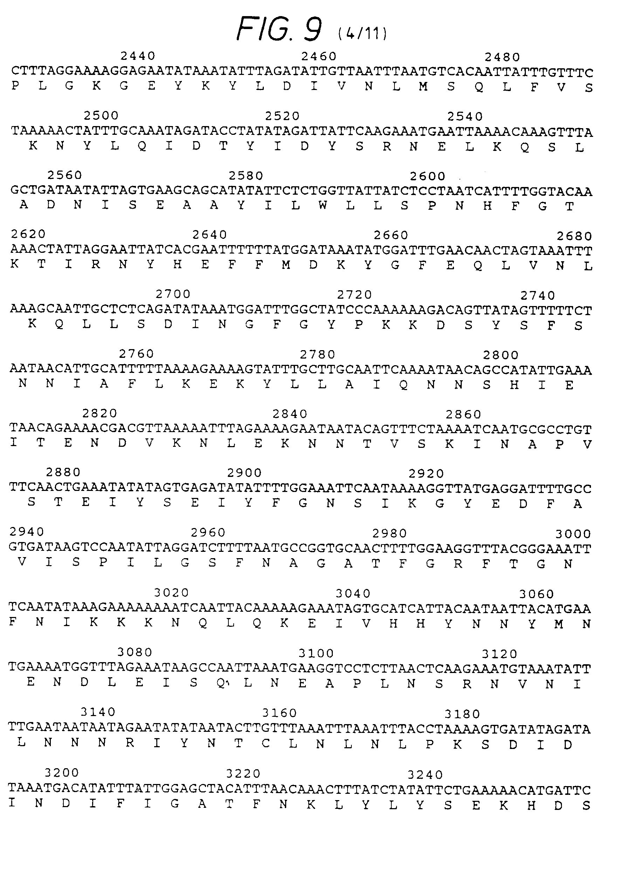

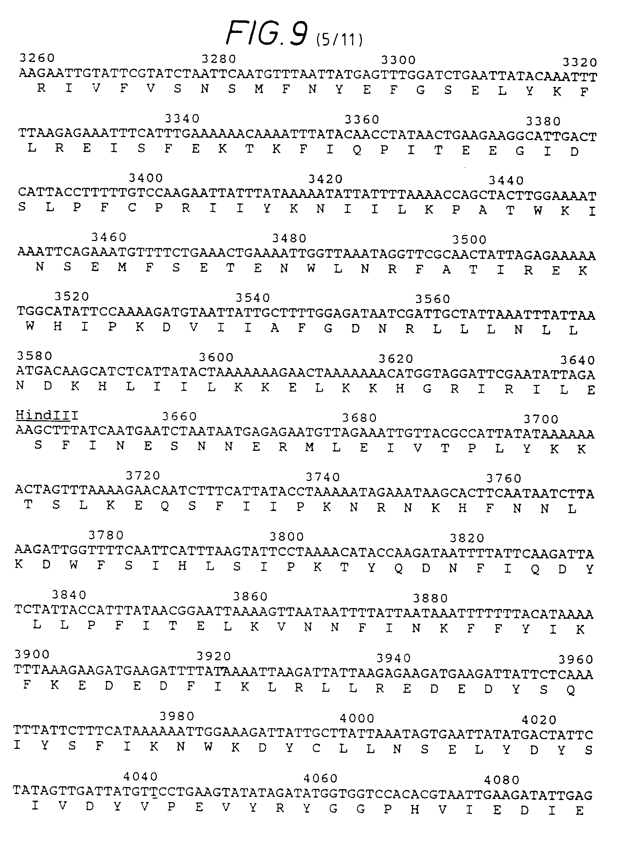

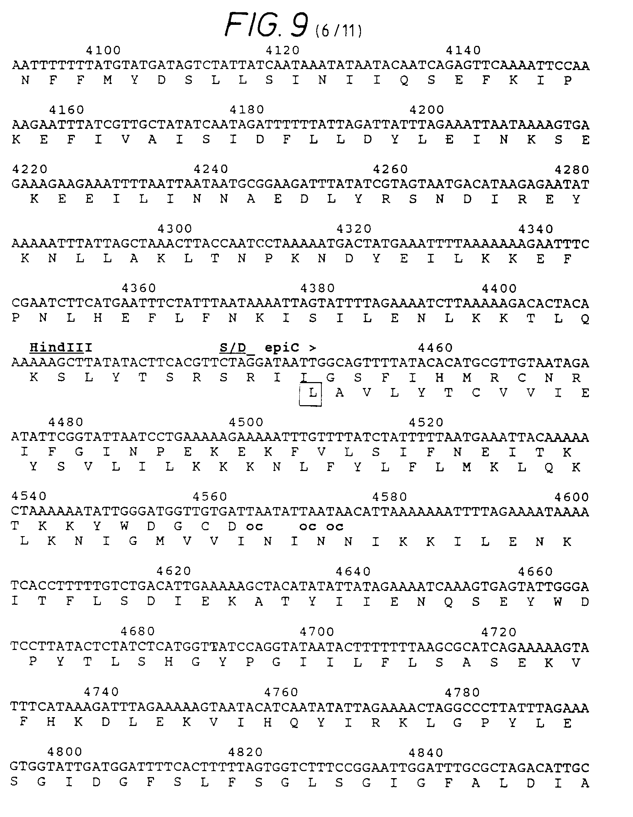

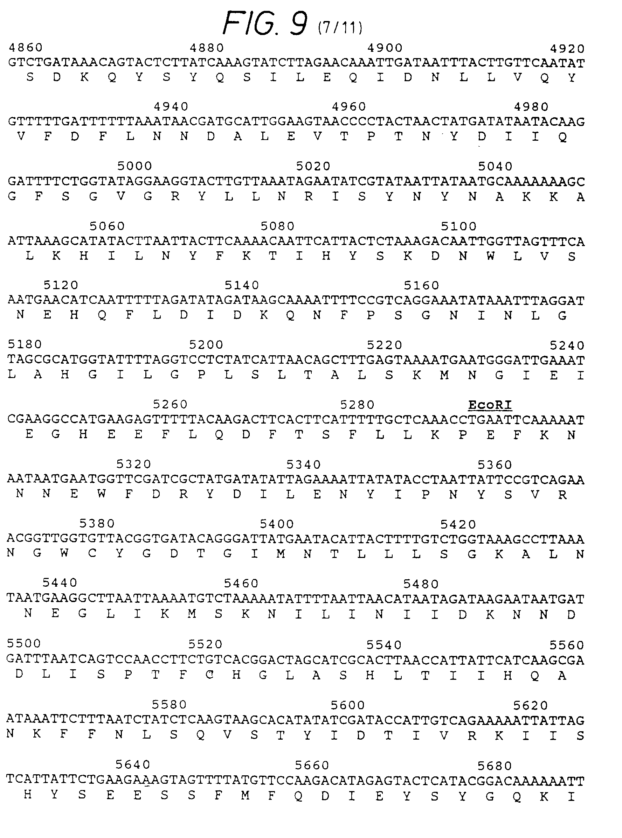

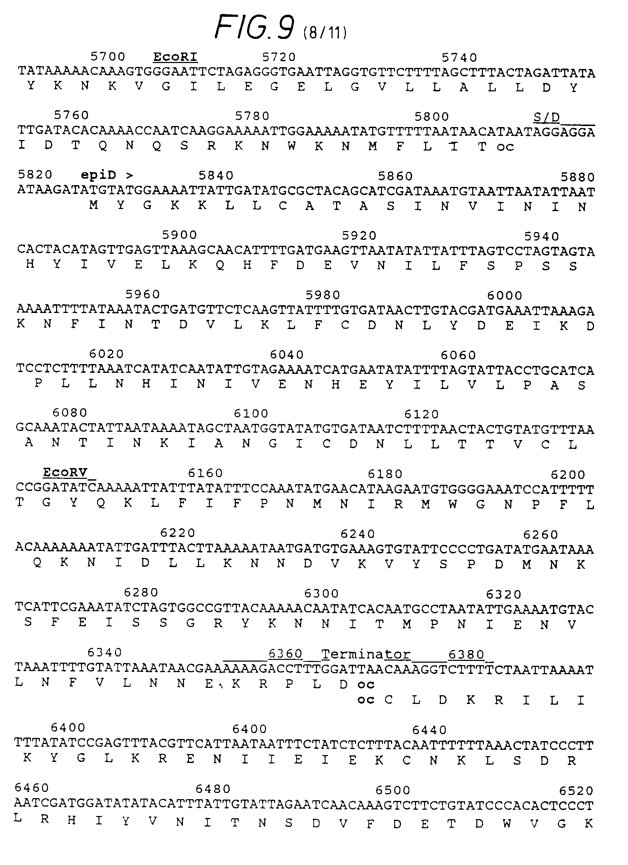

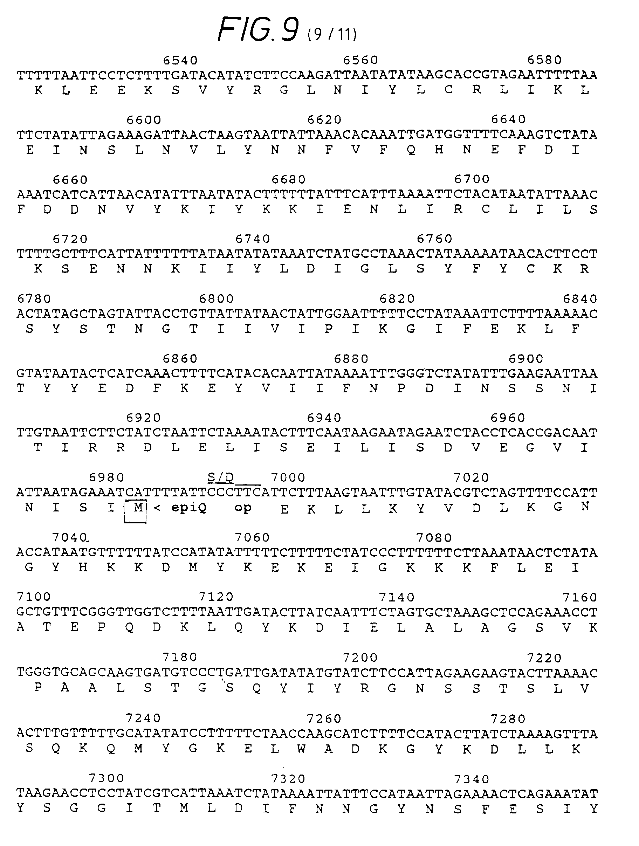

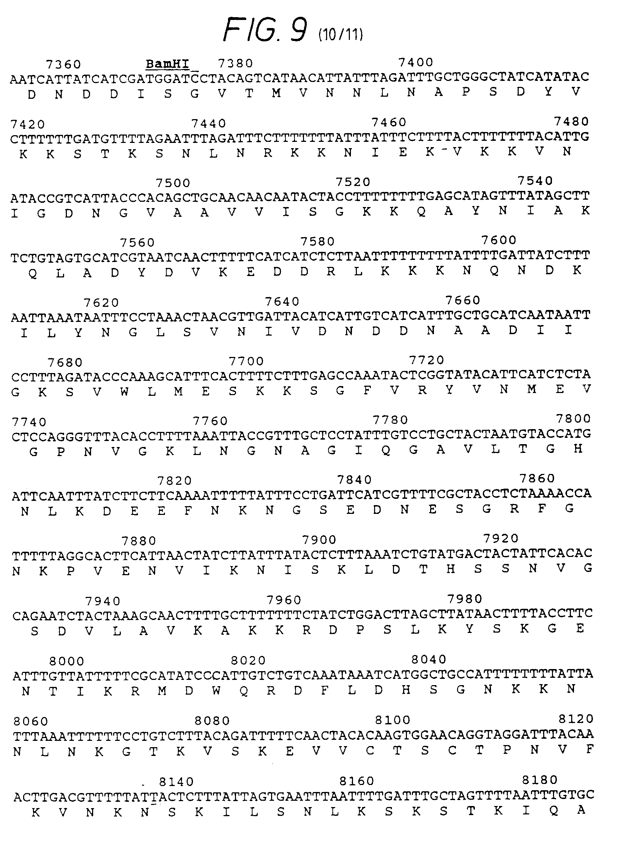

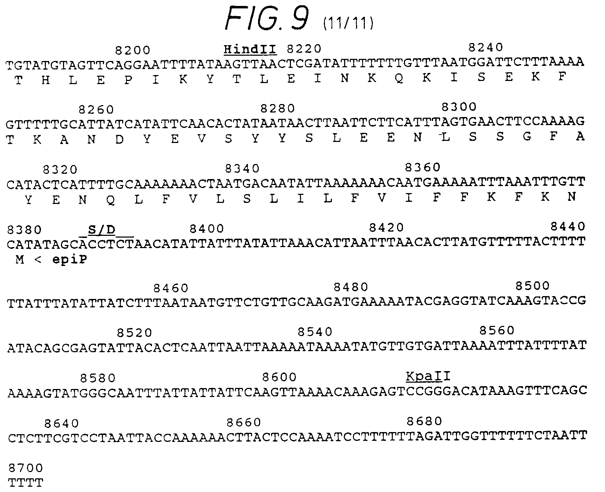

Figure 9 is a nucleotide sequence of the BglII/HpaII fragment of pTü32 containing reading frames epiA, B, C, D, P, Q, Y', and Y'' and the deduced amino acid sequences of the respective proteins. S/D sequences and termination structures are overlined. IR indicates inverted repeats. The start of the open reading frames of epiY, epiA, epiB, epiC, epiD, epiQ and epiP are indicated by bold letters. The N-terminal amino acid residues (possible translational start sites) are boxed.

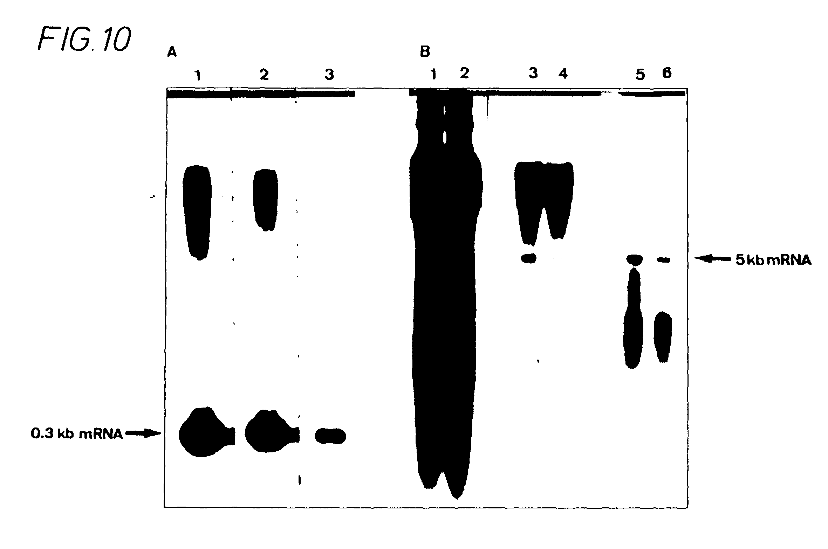

Figure 10 shows the results of a Northern blot analysis of epiA (10A) and epiB (10B) expression in *S. epidermidis*, in which total RNA (40µg, lanes 1, 3, and 5, or 20 µg, lanes 2, 4, and 6) were separated on 1.2% agarose gels and hybridization was performed with an antisense RNA probe (SP6 transcript. Filters were washed with increasing stringency; lanes 1, 2:1xSSC, 0.1% SDS, exposition time, 4 h, lanes 3, 4:0.5xSSC, 0.1% SDS, exposition time 16 h; lanes 5,6:0.1x SSC, 0.1% SDS, exposition time, 3 days). The positions of 235 and 165 RNAs were used as a size standards.

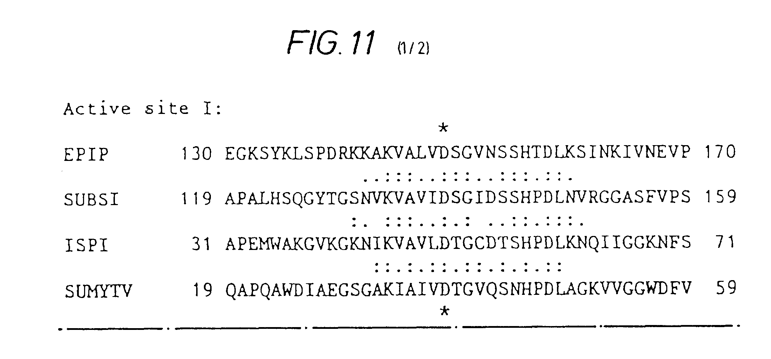

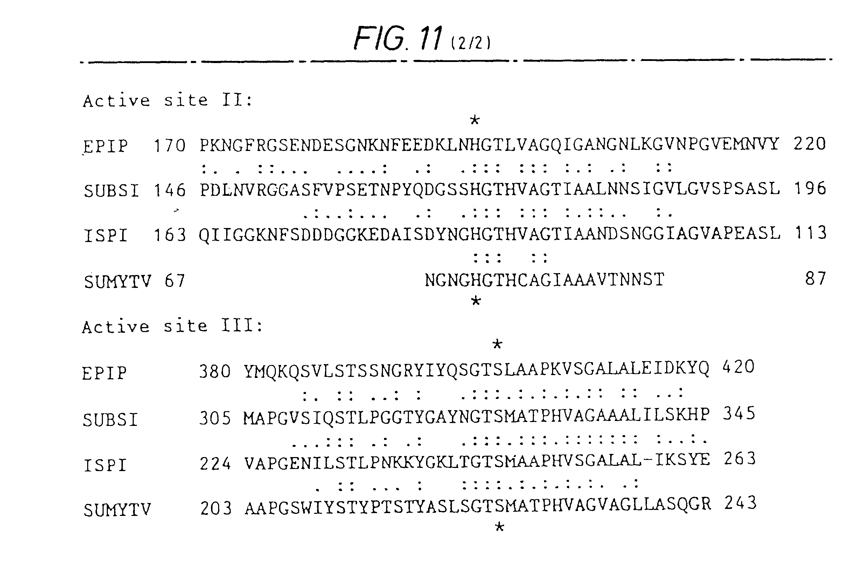

Figure 11 shows sequence homologies between EpiP and different serine proteases at the active sites (SUBSI, subtilisin I168 precursor of *B*. *subtilis* (Terzaghi *et al*., *Appl. Microbiol*. *29*: 807-813 (1975); major intracellular serine protease from *B. subtilis* (Maniatis *et al*., *Molecular Cloning*. *A Laboratory Manual*; 2nd ed. Cold Spring Harbour Laboratory Press (1990); SUMYTV, thermitase from *Thermoactinomyces vulgaris* (Stahl *et al*., *J. Bacteriol 158*: 411-418 (1984)). The strongly conserved asparagine (asp), histidine (his), and serine (ser) residues are marked by asterisks. Similar amino acid residues are indicated by points and identical amino acid residues by colons.

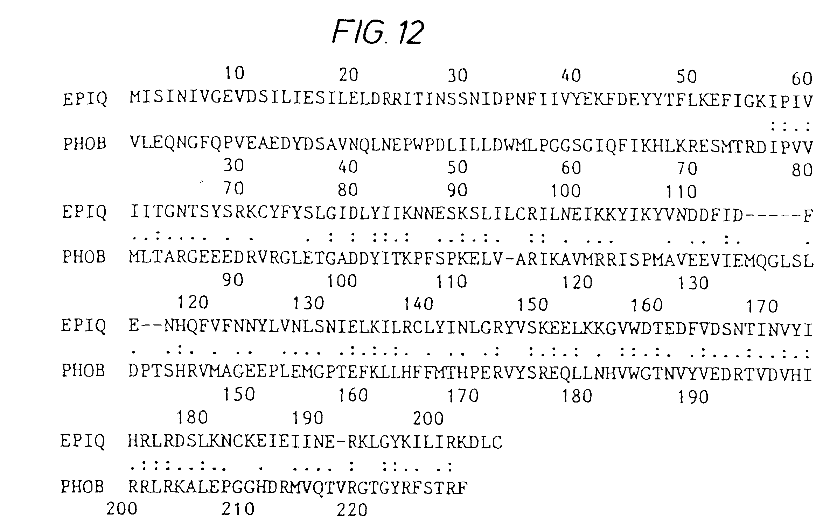

Figure 12 shows sequence homologies between epiQ and PhoB (Makino *et al*., *J. Mol. Biol*. *190*:37-44 (1986)). Similar amino acid residues are indicated by points and identical amino acid residues by colons.

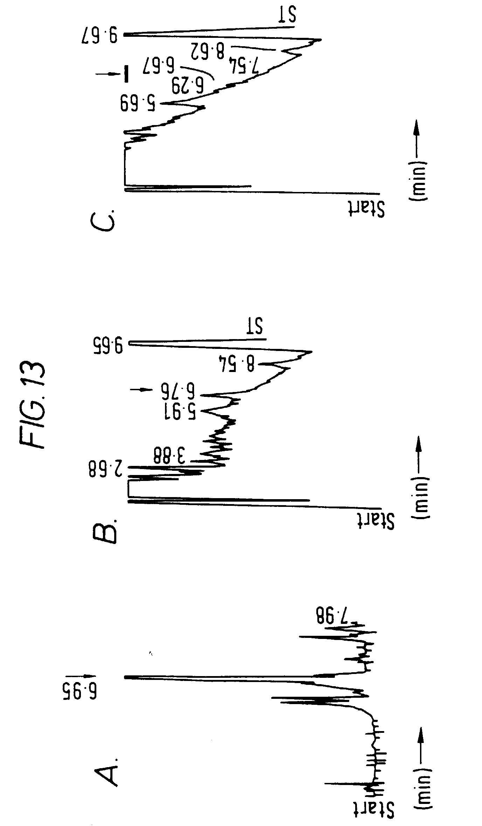

Figure 13 is a HPLC elution profile of epidermin which was produced in *S. carnosus* TM300.
- 13A:: Elution profile of epidermin standard substance (6.75 min, indicated by an arrow).
- 13B:: Elution profile of epidermin standard substance (6.75 min, indicated by an arrow) isolated from culture filtrates of *S. carnosus* TM300 pTepil4. Culture filtrates were adsorbed to XAD 1180, eluted with methanol and finally concentrated by evaporation.
- 13C:: Elution profile of untransformed *S. carnosus* TM300 culture filtrate treated as in 13B. The solid line indicates the elution region of epidermin.

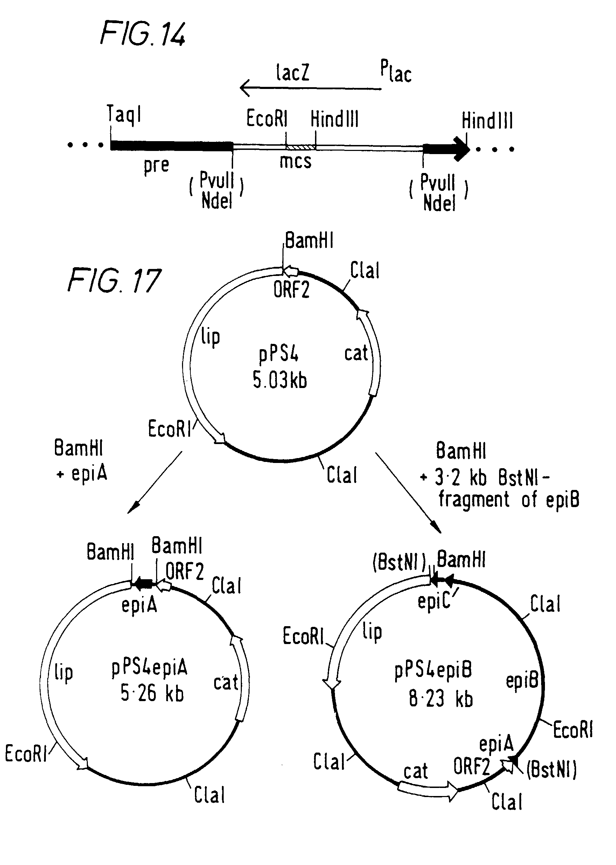

Figure 14 shows the construction of pT181mcs. The PvuII³⁰⁹-PvuII⁶³¹ fragment of pUC19, part of lacZ and the multiple cloning site (mcs), was inserted into the single NdeI site within pre of pT181 (Gennaro *et al.*, *J. Bacteriol.* 169:2601-2610 (1987); Kahn *et al.*, *Plasmid* 10:251-259 (1983)) by blunt-end ligation. lacZ is in the opposite orientation to *apre.* Black bar, interrupted *pre*; open bar, inserted pUC19 fragment.

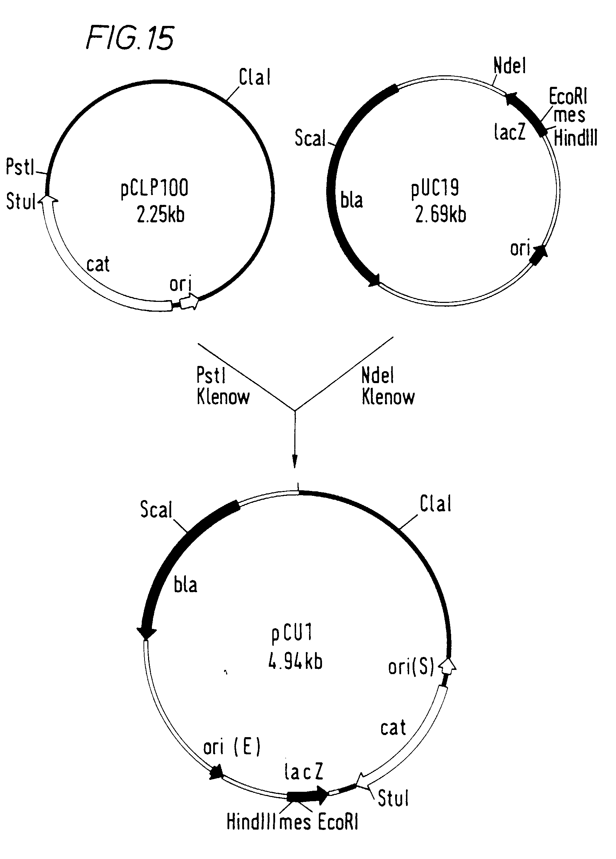

Figure 15 shows the construction of pCU1. PCLP100 is a derivative of pC194 (Horinouchi *et al., J. Bacteriol.* 150:815-825 (1982)) containing a single PstI site which was generated by opening pC194 at the HindIII site, deleting the ends with Ba131 (approximately 950 bp) and inserting a PstI-linker by blunt-end ligation. PCUI was then generated by blunt-end ligation of pCPL100 and pUC19 (Vieira *et al.*, *Gene 19*:259-268 (1982)) via the single PstI and NdeI sites, respectively. The multiple cloning site (mcs) in front of lacZ was used for cloning various epi gene-containing fragments. This shuttle vector replicates both in staphylococci and *E. coli.*

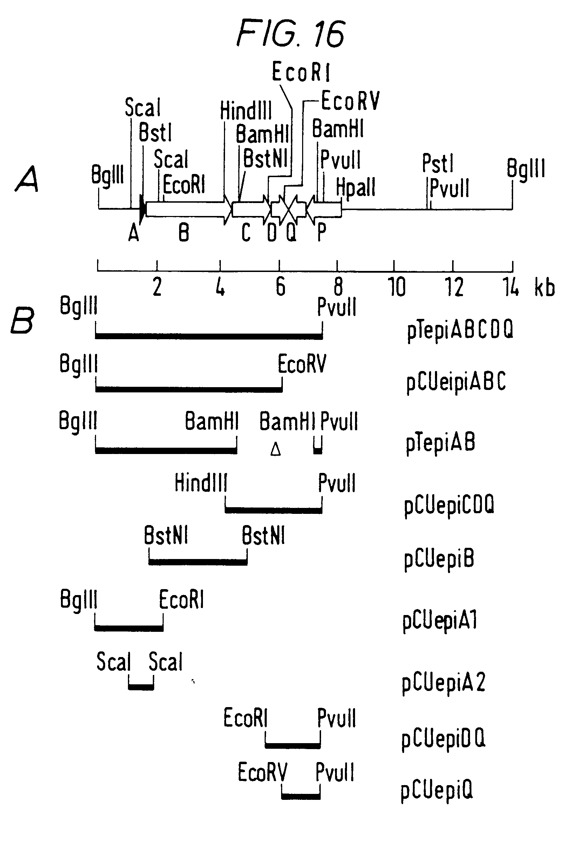

Figure 16 shows:
A) the generation of pTepi14 by cloning the 14 kb BglII fragment of pTü32 in pT181 mcs. This fragment containing the entire genetic information necessary for epidermin production in *S. carnosus*. The indicated ORFs and their transcriptional directions (indicated by arrows) are deduced from the DNA sequence. epiA, the structural gene, is presented by the black arrow.
B) various pTepil4 DNA fragments subcloned into pT181mcs (pT...) or pCU1 (pCU...). The respective plasmids were used to complement the S. *epidermidis* Epi mutants. The complete ORFs present in the plasmid are indicated.

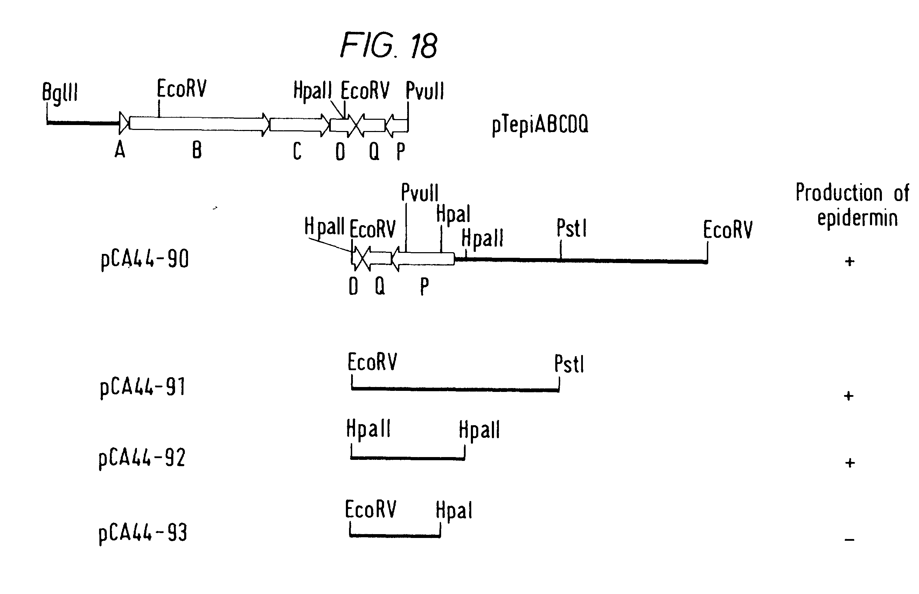

Figure 17 shows the construction of pPS4epiA and pPS4epiB. pPS4 is a derivative of pLipPS1 (Liebl *et al., Mol. Gen. Genet. 204*:166-173 (1986)). A single BamHI site was inserted after a strong staphylococcal promoter. Cloning of genes into the BamHI site under the control of the ORF2 promoter normally leads to good expression in staphylococci. epiA was PCR-amplified and contained flanking BamHI sites. The 3.2 kb BstNI fragment containing epiB was inserted into the BamHI site by blunt-end ligation. The respective EMS-mutants were complemented only when epiA and epiB were under the control of the ORF2 promoter. *lip*, lipase gene; *cat,* chloramphenicol acetyl transferase gene; ORF2, *S. carnosus*-specific truncated ORF.

Figure 18 shows the complementation of epidermin production in *S. carnosus* (pTepiABCDQ) by flanking DNA fragments. The fragments were subcloned into the compatible plasmid pCA44.

### Description of the Preferred Embodiments

Broadly speaking the present invention provides in one aspect a bacterial host containing a plasmid, wherein said plasmid codes for a polypeptide which is not normally produced by said host, and wherein said host during cultivation provides a multi-enzyme complex whereby a polypeptide is produced which contains at least one dehydroamino acid and/or at least one lanthionine bridge, said produced polypeptide being foreign to said host

A suitable multi enzyme complex is one which is capable of effecting at least one of the following operations, namely water elimination and sulphide bridge formation; the complex may also effect decarboxylation and double bond formation.

Suitable hosts for carrying out the process of the present invention are those which, without modification of their genetic material, are capable of producing polypeptides containing a dehydroamino acid residue and/or lanthionine bridge and/or a methyl lanthionine bridge. Examples of such hosts are *Streptococcus lactis, Bacillus subtilis, Streptomyces cinnamoneus, Streptomyces sp. Streptoverticullum griseoverticillum, Staphylococcus epidermidis*, *Staphylococcus epidermidis* strain 5, *Staphylococcus gallinarum* and mutant strains thereof, e.g., a mutant strain of *S. epidermidis* DSM 3095 which is incapable of producing epidermin.

Strains which are of special interest are *Staphylococcus gallinarum* (F16/P57) Tü 3928 which has been deposited with the Deutsche Sammlung von Microorganismen under the terms of the Budapest Treaty on 18 May 1988 and has received the depository number Tü 3928 in DSM 4616 and *Staphylococcus epidermidis* DSM 3095 which was deposited by the present applicants with the Deutsche Sammlung von Microorganismen under the terms of the Budapest Treaty on 26th October 1984.

In order to transform a suitable host, a suitable plasmid may be modified by known genetic engineering techniques.

Desirably a plasmid from a host which produces a polypeptide containing at least one dehydroamino acid residue and/or at least one sulfide bridge is treated by modifying or replacing the gene coding for a pre-polypeptide to provide a plasmid coding for a polypeptide foreign to said host and then transforming said host with the altered plasmid.

Any of a variety of methods may be used to replace or modify a gene coding for the pre-polypeptide.

DNA coding for the pre-polypeptide sequence of the desired compound can be prepared by chemical synthesis. Suitable chemical syntheses have been disclosed in *Anal*. *Biochem*. *121*, 365 (1982). The known techniques allow the preparation of polynucleotides, e.g., of up to 60 to 100 bases to be prepared.

Suitable protected nucleotides can be linked by the phosophotriester method Agarwal *et al*., (*Agnew*, *Chem*. *84*, 489 (1972)), the phosphotriester method (Reesem., *Tetrahedron 39*, 3, (1983)) or the phosphitetriester method (Letsinger *et al*., *J*. *Am*. *Chem. Soc*. *98*, 3655 (1976)) or the phosphoramidite method. The solid phase method allows for simplification of the synthesis of the polynucleotides.

The double stranded DNA can be constructed enzymatically from chemically prepared short but overlapping segments.

For example, overlapping polynucleotide sequences from both DNA strands can be used, which are held together in the correct conformation by base pairing and are then chemically linked by the enzyme DNA ligase (Khorana *et al*., *J*. *Biol*. *Chem*. *251*, 565 (1976)).

Another possibility comprises incubating in each case one polynucleotide sequence from the two DNA stands with a short overlapping segment in the presence of the four required deoxynucleoside triphosphates with a DNA-polymerase, for example, DNA-polymerase I, the Klenow fragment of polymerase I or T4 DNA-polymerase, or with reverse transcriptase. The two polynucleotide sequences are thereby held together in the correct arrangement by base pairing and are supplemented with the required nucleotides by the enzyme to give a complete double-strand DNA (Narany *et al*., *Anal*. *Biochem*. *121*, 365 (1982)).

Another suitable method for obtaining the DNA coding for a polypeptide comprises isolating the DNA from the genomic DNA of a tissue or cell culture or microorganism, lysing the cells e.g. with SDS or proteinase K, or if desired mechanically, and deproteinising the DNA by repeated extraction with phenol.

The RNA can be preferably digested with RNase. The obtained raw DNA is partially digested with suitable restriction enzymes e.g. HaeIII and AluI and fragments isolated and multiplied in a suitable phage or cosmid, e.g. in charon 4A or EMBL-3 phage and assayed for the desired sequences e.g. with a radioactively labelled DNA probe.

The DNA coding for a desired polypeptide can also be obtained by reverse transcription of isolated mRNA into cDNA. This may be the preferred method if the DNA structure is not known. In this method the DNA is obtained from genomic DNA in a cDNA library via the mRNA. The cDNA library comprises the genetic information which is complementary to the mRNA isolated from cells.

To obtain a cDNA library, the mRNA is isolated from cells expressing the desired basic (possibly unmodified) protein. This mRNA is converted into double stranded cDNA.

Standard methods well known in the art are applied in the preparation of mRNA. The cell membrane is broken and the cell content released from which the mRNA is isolated. The cell membrane is preferably broken by physical methods or lysis with detergents such as SDS, guanidine thiocyanate, definite salt conditions or homogenization, preferably by mixing. The mRNA is isolated by the standard methods of phenol extraction, ethanol precipitation, centrifugation and chromatography, preferably a combination of several methods. Centrifugation is preferably done over gradients, for example over a CsCl gradient. For chromatography, preferably columns are used, especially oligo-dT columns.

The total mRNA can be converted directly into Ds-cDNA following the methods of the art. Preferably the mRNA coding for a desired polypeptide is further enriched using several techniques, such as electrophoresis, chromatography and centrifugation, preferably sucrose gradient centrifugation.

Fractions containing mRNA coding for a desired polypeptide can be detected by various methods, such as *in vivo* or *in vitro* translations, followed by detection of a relevant activity or, when the nucleotide sequence is known, by hybridization with an oligonucleotide probe.

*In vivo* translation systems can be prokaryotic or eukaryotic systems. A preferred *in vivo* translation system is the Xenopus laevis oocyte system (see Maniatis *et al*., *Molecular Cloning A Laboratory Manual Cold Spring Harbor Laboratory* (1982)). In vitro systems are, for example, wheat germ and rabbit reticulocyte lysates, both of which are commercially available.

From any pool of mRNA derived from unfractionated or fractionated mRNA, ds-cDNA can be obtained by the well known methods of the art (preferred general methods are described in Maniatis *et al*. (*supra*), Okayam and Berg, *Molecular and Cell Biology 2*, 161-170 (1982) and Heidecker, *Nucleic Acid Research 11*, 4891-4906 (1983)). In general, the mRNA is converted first to ss-cDNA using reverse transcriptase or DNA-polymerase I (Klenow fragment). Two methods are alternatively used for priming the synthesis of the ds-cDNA. The first method was the natural loop formation of the ss-cDNA. The second method is that of tailing the ss-cDNA with a homopolymeric tail such as poly-dC or poly-DT.

The mRNA fraction of which the corresponding polypeptide shows the highest activity in the detection system is transcribed into the complementary cDNA by methods well known in the art. The mRNA and oligo-dT as a primer are mixed, dNTPs are then added as starting material and the synthesis of the cDNA-mRNA hybrid molecule is realized by the enzyme reverse transcriptase. The RNA molecules are degraded by addition of NaOH. DNA polymerase is admixed, preferably the Klenow fragment of the DNA polymerase I, and the mixture is incubated at a suitable temperature, preferably 12-15°C. The mixture is incubated with nuclease S1 and the ds-cDNA corresponding to the mRNA coding for a desired polypeptide is obtained.

For amplification the obtained ds-cDNA can be spliced into suitable vector e.g. the plasmid pUC-KO and the obtained hybrid vector multiplied by use of a suitable host, e.g. *E. Coli* HB101. Reisolation of the hybrid vectors, and recovering the isolated cDNA therefore allows a sequence determination of the DNA coding for a desired polypeptide.

### Preparation of a Hybrid Vector

A hybrid vector of the invention can be prepare by splicing a DNA coding for a polypeptide of the desired sequence into a suitable vector.

Suitable vectors are carriers for integrated passenger DNA, which can be used to transform a host microorganism.

Suitable as vectors are plasmids derived from microorganisms which in an untransformed state produce polypeptides which contain dehydroamino and/or sulfide groups. Suitable vectors carry the insert DNA at a defined position.

In general, such vectors may contain a replicon and a control sequence, i.e. a promoter, which are derived from the host cell or a species compatible with the host cell in which they are used. The vector ordinarily carries a replicon site and may contain sequences (marker genes) which are capable of providing phenotype selection in transformed cells. Suitable marker genes may provide antibiotic resistance or resistance to heavy metals or they may complement a genetic defect of the host. Further useful sequences in such vectors are enhancer and activator sequences.

One suitable starting vector is a 54 Kbp plasmid pEpi32 from the strain *Staphylococcus epidermis* DSM 3095. This plasmid, which is characterized below, contains the *epi*A gene encoding for a 52-prepeptide, which is processed to a tetracyclic 21-peptide amide antibiotic. A vector carrying a passenger DNA is designated a hybrid vector.

The desired DNA is spliced into the starting vector by conventional methods.

A starting plasmid for example can first be linearised by a suitable restriction enzymes, e.g. the plasmid pEpi32 by HindIII, BamHI and EcoRI, then d/G-tailed in the presence of dGTP and the terminal deoxynucleotidyl transferase. The double stranded cDNA insert is dC-tailed in the presence of dCTP and terminal deoxynucleotidyl transferase. Combining both cDNA and vector results in the hybrid vector. Bacteriophages, such as lambda, are preferred for constructing genomic libraries. The lambda cloning systems are described by Maniatis (*supra*). The suitable vector DNA is digested to completion with the appropriate restriction enzyme, and the left and right arms are separated from the central fragments by velocity gradient centrifugation or gel electrophoresis. Another method is to digest parts of the stuffer fragments with restriction enzymes which lack recognition sites in the left and right arms. The isolated genomic DNA can be partially digested to fragments of 13-20kb in length. Afterwards the arms are ligated with the fragments of foreign DNA having termini compatible with those of the arms.

The appropriate DNA insert is recloned from the original vector used for the original cloning, into a suitable expression vector. To this end appropriate restriction enzymes are used, possibly in combination with oxonucleones, to produce the desired DNA fragments.

The DNA insert may be subcloned into a multiple site of a suitable well known plasmid vector e.g. derivatives of pC194, pTI81 and pUB110 at the restriction sites HindIII/BamHI/EcoRI.

The method of the invention can thus be used to prepare derivatives of known peptides and hormones, in which a cystein residue in the unmodified peptide is replaced by sulfide-bridged amino acids and serine and threonine replaced by corresponding dehydroamino acid residues.

These fragments are integrated into an appropriate expression vector by using the cohesive ends directly or by the addition of appropriate chemically synthesized oligonucleotide bridges. For the modification of the ends for example HindIII and BgLII can be used. The method is not limited to any special restriction enzymes. Any desired link can be made between the expression vector and the DNA insert using suitable restriction enzymes in combination with chemically synthesized oligonucleotides.

Appropriate DNA inserts can also be obtained which code for polypeptide having site directed mutagenesis.

A variety of methods may be used to induce mutations of underlying DNA so as to prepare the desired mutants.

One method may comprise first inserting a fragment of a native or basic gene, containing sequences coding for the region to be mutated, into the replicative form of a phage, e.g. phage MI3mp8 to form MI3mp8PA. A synthetic oligonucleotide, complementary to the inserted sequences but containing one or more nucleotidetriplets which code for the amino acid to be substituted, is then annealed to the single stranded form of MI3mp8A to form a double stranded region. This region serves as a primer for DNA polymerase I synthesis of the remaining complementary strand. After replication and identification, the mutant sequence may be further modified or used to construct a suitable vector for expressing the mutated polypeptide.

In the work carried out on epidermin a wobbled DNA probe 5'-GTG(A)CAT(G/A)ATG(A)AAT(C)TT-3' deduced from a suitable pentapeptide segment of the proposed pre-sequence of epidermin LysPheIleCylThr was prepared. This DNA probe was hybridized against plasmid DNA from *S. epidermin* DSM 3095.

Restriction analysis of the isolated plasmid reveals seven DNA fragments with EcoRI (16, 11, 10, 6.5, 5.5., 3.5 and 2.5 kbp), nine DNA fragments with HindIII (17, 14, 10, 5.3, 2.8, 1.8, 0.8, 0.6 and 0.5 kbp) and five DNA fragments with BamHI (20, 19, 10, 3 and 1 kbp).

A 5.4 kbp HindIII fragment was subcloned and subjected to rehybridization whereby the structure gene epiA was located within a 2.2 kbp EcoRI/BglII fragment.

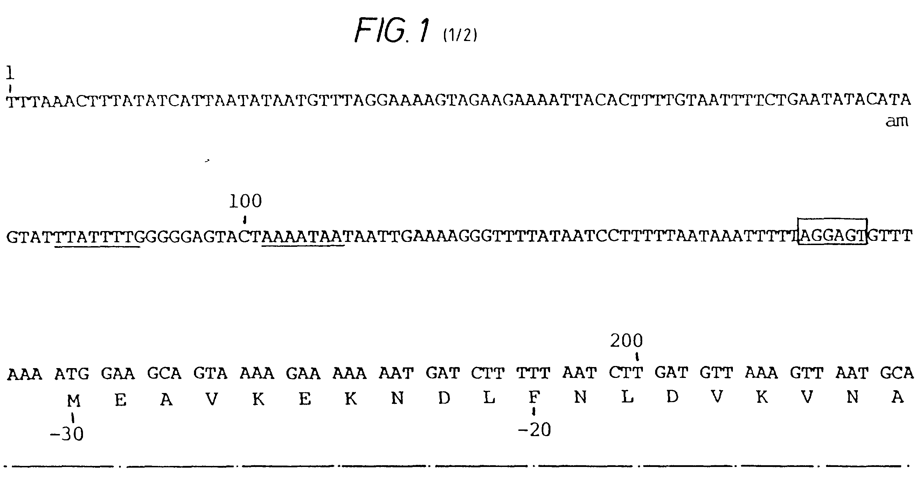

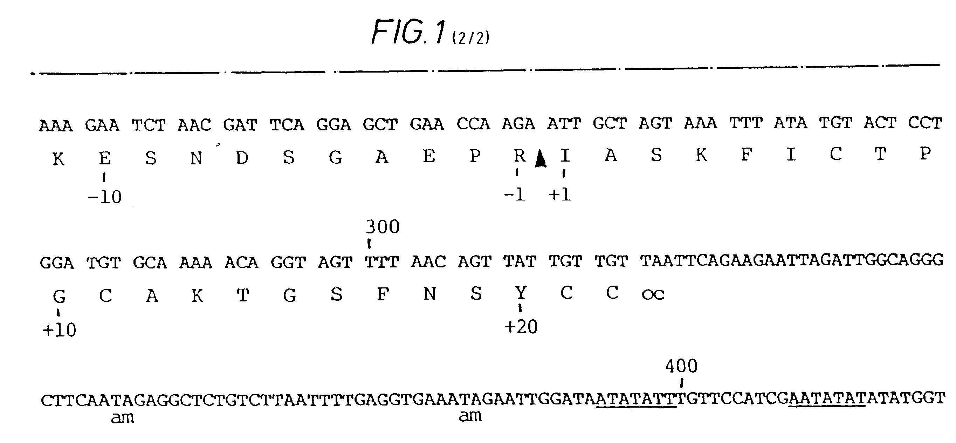

As a mixture of 24 different 14-mers was used as a hybridization probe. The probe was applied in a 30-fold excess as a sequencing primer in accordance with the techniques described in Novick *et al. Ann. N.Y. Acad. Sci. 182*, 279-294 (1971), Southern, *J. Molec. Biol. 98*, 503-517 (1975) and Heinrich *et al*., *Molecul*. *gen. Genet. 209*, 563-569 (1987). The peptide sequence of epidermin allowed identification of the open reading frame. A single methionine codon is in appropriate distance to a Shine-Dalgaro sequence. The structural gene of pre-epidermin terminates at the TAA stop codon, hence pre-epidermin consists of 52 amino acids (Figure 1) and it is processed to the epidermin between Arg⁻¹ and Ile⁺¹. Thus, as can clearly be seen, pre-epidermin is not a degradation product of a larger protein but is coded by a distinct structural gene.

Thus, it is apparent that, unexpectedly, the precursor protein of the antibiotics are coded by distinct structural genes.

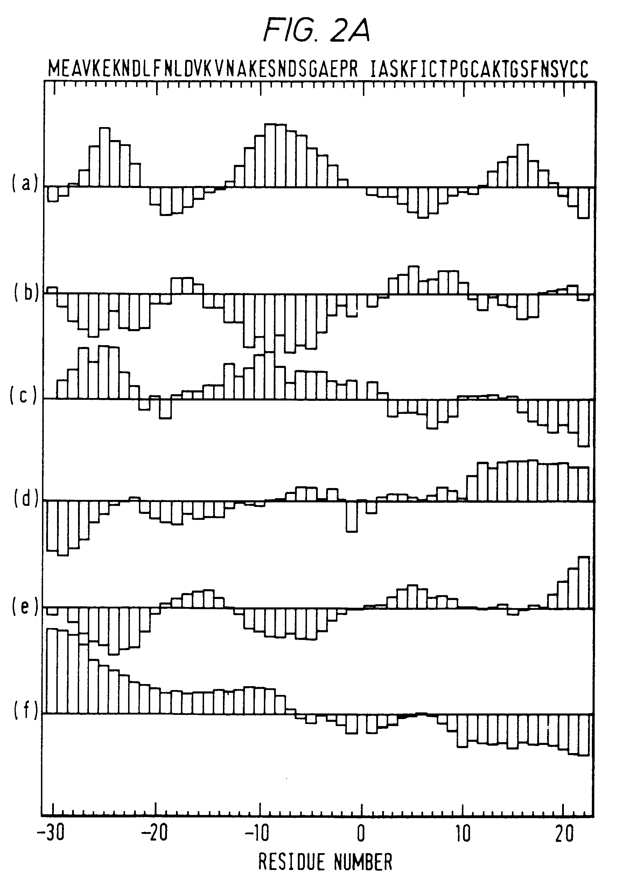

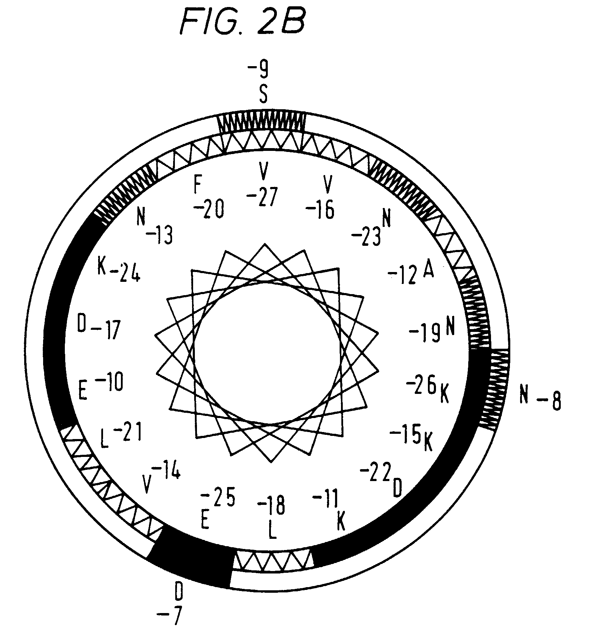

A combination of prediction profiles for secondary structure (α, β, turns), flexibility, hydropathy, hydrophilicity (Figure 2A) and helix wheel plot were made using a Hycon program (Figure 2B). A high α-helix probability is predicted for pre-epidermin -30 to -8 whereas the C-terminal part 1-22 which corresponds to pro-epidermin exhibits very high turn probability. Moreover, the prediction plot shows clearly, that the N-terminus -30 to -1 highly hydrophilic, whereas the C-terminal part is more lipophilic. The N-terminal part -30 to -8 seems to fold partially into an amphophilic α-helix.

The N-terminal segment of pre-epidermin -30 to -1 does not contain any cysteine residues, whereas the C-terminal segment 1-22 contains the four cysteine residues, involved in sulphide bridge formation. Sequence -30 to -1 included many cleavage sites for endoproteases whereas even in the pre-epidermin state, sequence 1-22 is highly resistent to proteolytic degradation.

The mature antibiotic can only be attacked by trypsin at Lys in position 13. The processing site Arg⁻¹-Ile⁺¹ is hydrophilic and accessible, due to the turn forming Pro⁻² residue.

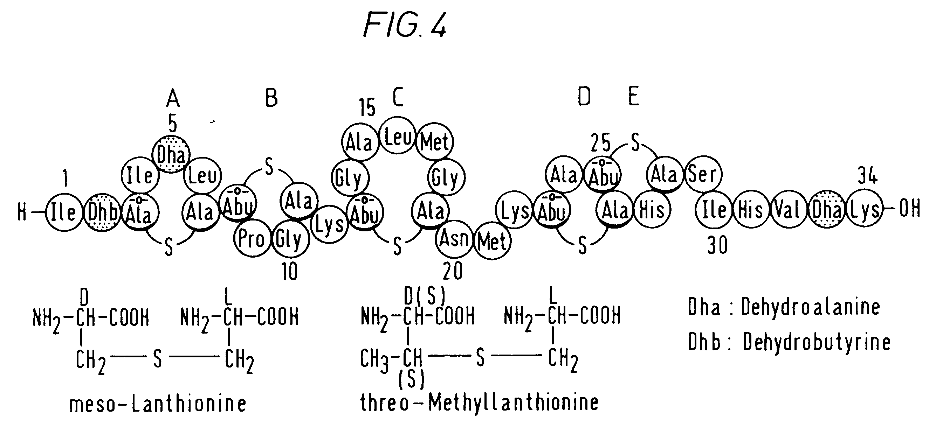

The various enzymatic reactions which occur in the production of the antibiotics such as epidermin include modifications of the propolypeptide part 1-22; cleavage of the N-terminal prepeptide fragment -30 to -1 and secretion of the matured antibiotic (see Figures 3 and 4).

The enzymatic modifications occur before cleavage of the prepeptide fragment. Enzymatic modification includes the elimination of water from Ser and Thr residues in position 5, 16, 19 and 8, 14 respectively to form dehydroalanine and dehydrobutyrine residues. Addition of thiol groups of Cys residues in position 2, 11, 21 and 22 to the C=C double bonds, also occurs, yielding the meso-lanthionine or (2S 3S, 6R)-3-methyl-lanthionine bridges. In addition, decarboxylation of residue 22 and double bond formation yields the C-terminal S-(2-aminovinyl)-D-cysteine. The reaction of C-terminally situated cysteine thiol groups with N-terminally located dehydroamino acids occurs with complete stereo-specificity in epidermin, nisin and subtilin. Accordingly, during modification these elimination-addition reaction imply a reversal of configuration of the Cα carbon atoms at pre-epidermin residues L-Ser and L-Thr to give D-configured Cα atoms. On the other hand, the L-configuration of the cysteine halves is still maintained.

The four sulphide rings are also formed, subsequently at the same catalytic site, which is supported by the interaction with the N-terminal amphophilic α-helix. Only Thr⁺¹⁴ dehydrates without finding a cysteine. This position (Lys⁺¹³-Dhb⁺¹⁴) constitutes the enzymatic cleavage site at which trypsin inactivates the antibiotic epidermin. During sulphide ring formation C-terminal rigidity and hydrophobicity increases and may favor interaction of pro-epidermin with the lipid bilayer and may induce translocation.

Finally, the hydrophilic α-helical N-terminus -30 to -1 is cleaved by a specific protease at the characteristic cleavage site described above.

Using the techniques described above plasmids coding for lantibiotics can be modified either by mutation of the gene coding for the respective polypeptide or by replacement of such a gene by a gene coding for a different polypeptide and used to transform the original host or a different host, provided such host also, in its native state, is capable of expressing a lantibiotic.

Generally speaking, where the original functional gene codes for a pre-sequence, as discussed above for example in the case of epidermin, the DNA sequence coding for such a pre-sequence may be retained in the modified plasmid; in this case the DNA-sequence for the new, or mutated pro-polypeptide will be positioned directly upstream of the pre-sequence DNA similarly to the original pro-polypeptide sequence.

Cultivation of a bacterial host according to the present invention may be carried out under conventionally used cultivation conditions as described for instance in our co-pending British Patent Application No. 8811760.1 which was filed on 18th May 1988 and in European Patent Application Publication No. 0 181 578. Purification and isolation of the desired protein may also be carried out using the techniques or suitable modifications thereof described in the foregoing patent applications for epidermin and gallidermin, including the use of adsorbents, ion-exchange resins and if desired HPLC.

The process of the invention can be applied to the formation of novel compounds for experimental purposes, or to the formation of known compounds or derivatives of known compounds in new hosts. For instance a plasmid containing the gene coding for epidermin can be used to transform the species *Streptococcus lactis* to produce epidermin from that host, or the gene coding for Gallidermin (see our co-pending British Patent Application referred to above) can be used to replace the gene coding for the pro-polypeptide for epidermin in e.g. plasmid pEpi32 and used to transform *Staphylococcus epidermis* DSM 3095 to produce gallidermin from this host. Similarly other biologically active peptide derivatives containing dehydroamino acid residues and/or lanthionine bridges and/or methyllanthionine bridges can be produced, such as derivatives of hormones such as human insulin, oxytocin, vasopressin, peptide antibiotics, hormone inhibitors such as elastase inhibitor and fibrinolytically active agents such as human tissue plasminogen activator. Such derivatives, as well as retaining biological activity of the parent compound can have increased stability and improved half-lives.

Ideally the DNA coding for the desired pro-polypeptide should include codons for cystein and serine and/or for cysteine and threonine for the formation of thioether bridges.

For relatively short chain polypeptides these respective codons should normally be no more than eight and preferably no more than six codons apart, inclusive, although it is envisaged that, depending upon the steric conformation of the final polypeptide molecule much greater spacing is possible.

In respect of the formation of dehydroamino acids these will usually be derived from serine and threonine and, accordingly the DNA coding for the desired pro-polypeptide will include codons for such amino acids.

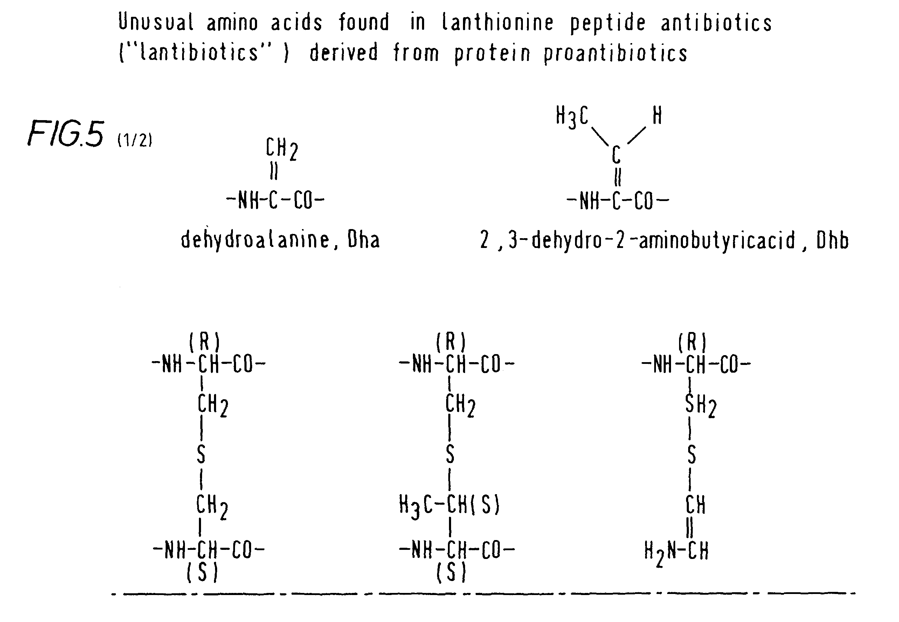

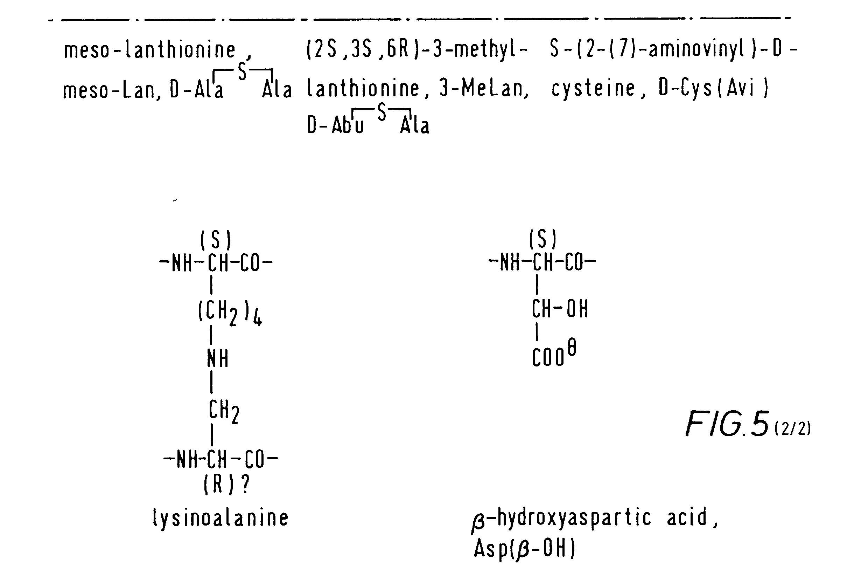

Amongst the unusual amino acids which may be present in a polypeptide produced according to the present invention are, dehydroalanine, 2,3-dehydro-2-aminobutyric acid, meso-lanthionine, (2S, 3S, 6R)-3methyl-lanthionine, S-(2-(Z)-aminovinyl)-D-cystein, lysinoalanine and β-hydroxyaspartic acid; the structure of these residues are shown in Figure 5

We have unexpectedly found that the multi enzyme complex responsible for the posttranslational modification of pre-epidermin is located on the 54kb plasmid pTü32 of *Staphylococcus epidermis* Tü 3298/DSM 3095. Plasmid pTü32 is sometimes also referred to herein as pEpi32.

The six genes (ORFs) responsible for the production of epidermin are designated herein epi A, B, C, D, Q and P and are clustered within 8kb and the proteins for which they code are designated Epi A, B, C, D, Q and P respectively; epi A encodes the 52 amino acid-long preepidermin. As described below, epi B, C and D are involved in the four enzymatic modification reactions (i) water elimination by a serine/threonine dehydratase, (ii) sulfur addition by a lanthinonine synthase, (iii) C-terminal decarboxylation by a cysteine decarboxylase and (iv) double bond formation. Epi P protein is believed to be responsible for cleaving the mature epidermin from the N-terminal leader peptide, based on its striking homologies with the essential domain of serine proteases (Koide *et al*., *J. Bacteriol*. *167*:110-116 (1986); Meloun *et al*., *FEBS Lett*. *183*:195-200 (1985); and Stahl *et al*., *J. Bacteriol 158*:411-418 (1984)) whilst Epi Q is believed to be a regulatory protein regulating epidermin biosynthesis, based on its distinct homology to the pho B gene of *E. coli* (Makino *et al*., *J. Mol. Biol.* 190:37-44(1986)), the fact that both proteins are of a similar size with 205 (epi Q) and 229 (pho B) amino acid residues, the observed homology of 24.2% extending over the 153 C-terminal amino acid residues and the hydrophilicity plots of both proteins.

As a result of the unexpected finding of the entire genetic information for the epidermin biosynthesis and the elucidation of the genes for the proteins epi B, C, D, Q and P, it is now possible to obtain the isolated DNA coding for the proteins, and to construct plasmids containing one or more of these genes so that upon cultivation of a host containing such plasmids one of these proteins alone or predetermined combinations of the proteins may be expressed and subsequently isolated.

The present invention therefore includes DNA sequences encoding respectively for the protein Epi B or Epi C, or Epi D, or Epi P or Epi Q. These sequences may be isolated DNA either single or double stranded, obtained by cleavage of and isolation from pTü32 in known manner or obtained by chemical synthesis or any other conventional procedure. The DNA may also be integrated in a plasmid, suitably an expression plasmid and under the control of a promoter regulator; such constructs when transformed into a suitable host which is then cultivated will express the protein Epi B, Epi C, Epi D, Epi P or Epi Q or combination of these proteins according to which DNAs were ligated into the plasmid. Alternatively plasmid pTü32 may be treated with suitable restriction nucleases to excise one or other of the DNA sequences, followed by religation after any necessary modification of the free ends of the digested plasmid, so as to create a modified plasmid containing DNA sequences coding for predetermined ones of epi B, C, D, P and Q.

A further variant comprises the substitution of the gene coding for epidermin in pTü32 with a DNA sequence coding for a predetermined amino acid sequence whereby cultivation of a suitable host with the modified plasmid will result in expression of a protein different from epidermin.

It is thus possible to substitute a DNA sequence encoding for gallidermin or mutant epidermin or other lantibiotic or other protein, for the epidermin coding sequence in pTü32 whereby the resulting plasmid can be transformed into a suitable host which may be a host normally incapable of producing a lantibiotic or any of the proteins Epi B, C, D, P or Q and to cultivate the host under conditions whereby the substituted DNA sequence and the genes epi B, C, D, P and Q are expressed, so as to obtain a protein which is gallidermin, mutant epidermin or other protein containing at least one structural feature of a lantibiotic.

Alternatively the genes coding for the proteins Epi B, C, D, P or Q may be inserted into a suitable vector, together with a DNA sequence encoding a predetermined amino acid sequence, the genes coding for the Epi proteins and the predetermined amino acid sequence being operably connected with suitable promoter regulator functions, the resulting plasmid being transformed into a suitable host which may be a host normally incapable of producing a lantibiotic or any of the proteins Epi B, C, D, P or Q, and the host cultivated so that the inserted genes cause the expression of a protein derived from said predetermined amino acid sequence but containing a lantibiotic structural feature, which protein may be gallidermin, epidermin, mutant epidermin, or another protein.

The present invention thus also includes within its scope DNA sequences capable of hybridizing, preferably under stringent conditions, with the DNA sequences described herein and coding for proteins having substantially the activity of the proteins Epi B, C, D, P or Q. Stringent hybridization conditions select for DNA sequences of greater than 85% or, more preferably, greater than about 90% homology. Screening of the cDNA library may be carried out under highly stringent conditions according to the method described in European Patent Application No. 88 119 602.9 and Kashima et al. (Nature 313:402-404 (1985)). The DNA sequences capable of hybridizing under stringent conditions with the DNA sequences disclosed in the present application may be, for example, allelic variants of the disclosed DNA sequences, may be naturally present in the particular microorganism but related to the disclosed DNA sequences, or may derived from other sources. General techniques of nucleic acid hybridization are disclosed by Maniatis, T. et al., In: Molecular Cloning, a Laboratory Manual, Cold Spring Harbor, NY (1982), and by Haymes, B.D. et al., In: Nucleic Acid Hybridization, a Practical Approach, IRL Press, Washington, DC (1985), which references are herein incorporated by reference. The proteins Epi B, C, D, P and Q are valuable and interesting new reagents potentially useful in the preparation of novel proteins or other substances containing structural features such as dehydroalanine, dehydrobutynine, meso-lanthionine, 3-methyl-lanthione, and S-(2-aminovinyl)-D-cysteine.

As such, they may be utilized as isolated proteins, or as chemical catalytic reagents in chemical synthesis procedures to investigate the extracellular processing of proteins by such enzymes.

The invention also relates to the proteins Epi B, C, D, P and Q in substantially pure form. By the term "substantially pure" is intended that the protein is free of the impurities that are naturally associated therewith. Substantial purity may be evidenced by a single band by electrophoresis.

The polypeptides of the invention may be isolated and purified from the above-described recombinant molecules in accordance with conventional methods, such as extraction, precipitation, chromatography, affinity chromatography, electrophoresis, or the like. Preferably, the polypeptides are produced as part of a fusion protein which further comprises an auxiliary protein. Such auxiliary which facilitates the isolation and purification of the polypeptide of interest. Such auxiliary proteins include, for example, typical secretion signals, the maltose binding protein from *E. coli*, or protein A. Methods for preparing fusion proteins comprising protein A, the purification thereof by immunoaffinity chromatography, and the cleavage thereof to release the protein of interest is taught, for example, in PCT Application Publication No. WO84/03103 (1984).

A necessary condition to permit cleavage of the fusion protein is that it contains a unique cleavage site which may be recognized and cleaved by suitable means. Such a cleavage site may be a unique amino acid sequence recognizable by chemical or enzymatic means and located between the desired protein and the auxiliary protein. Such a specific amino acid sequence must not occur within the desired protein or auxiliary protein. Examples of enzymatic reagents include proteases such as collagenase which may recognize the amino acid sequence NH₂-Pro-X-Gly-Pro-COOH, wherein X is an arbitrary amino acid residue, e.g. leucine; chymosin (rennin) which cleaves the Met-Phe bond; kallikrein B which cleaves on the carboxyl side of Arg in X-Phe-Arg-Y; enterokinase which recognizes the sequence X-(Asp)ₙ-Lys-Y, wherein n=2-4, and cleaves it on the carboxyl side of Lys; thrombin which cleaves at specific arginyl bonds. Examples of chemical agents which may be used to cleave the fusion proteins include cyanogen bromide which cleaves after Met; hydroxylamine which cleaves the Asn-Z bond wherein Z may be Gly, Leu or Ala; formic acid which in high concentration (^{∼}70%) specifically cleaves Asp-Pro.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

### EXAMPLES

### Example 1

### 1. Overproduction of gallidermin

A DNA fragment containing the open reading frame of gallidermin can be cloned in *Staphylococcus epidermidis* DSM 3095, the epidermin producing strain by using a medium copy plasmid such as pC194, pE194, pUB110, pT181 or pMK148 gallidermin. An increase of the gene doses usually correlates with an increase of product production; the correlation is not necessarily linear. High copy number plasmid derivatives of pC194 or pT181 can be used as cloning vehicles too.

### 2. Exchange of leader sequence

The leader-sequence of epidermin corresponding to amino acids -1 to -30, is involved in the secretion of epidermin. The sequence can be used to secrete other peptides in *S. epidermidis* such as gallidermin.

The leader-sequence DNA can be made portable by inserting respective linkers at the beginning and at the end of its sequence. Thus the leader sequence DNA can be isolated in large amounts from the plasmid and can be inserted at respective positions of other peptides and proteins. The leader-sequence DNA can also be produced by chemical synthesis.

### Example 2

### Production of Gallidermin using S. epidermis as host

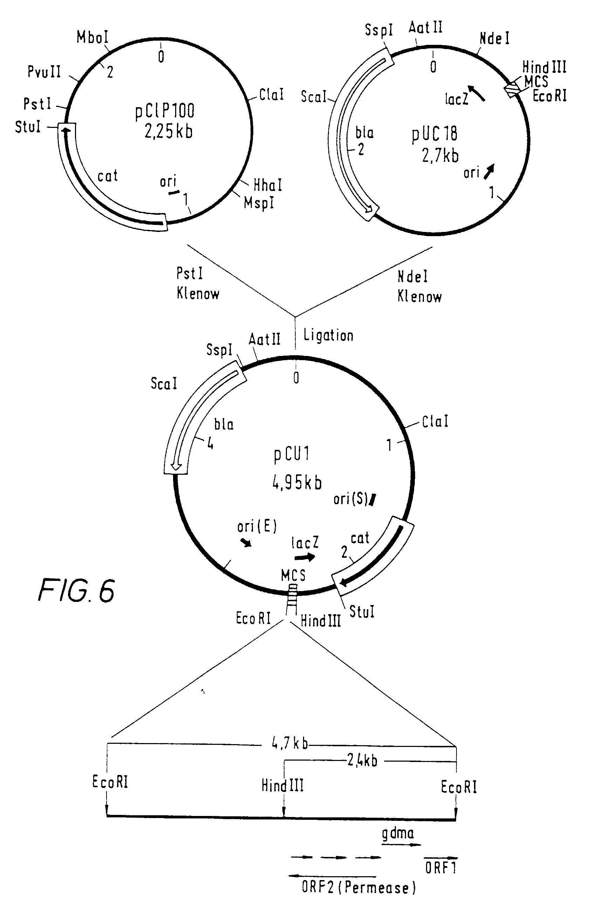

### 1. Preparation of plasmid (see Figure 6)

a) Plasmid pCUI was prepared by ligating Pst1 digested pCLP100 and Ndel digested pUC18 using Klenow as described in the thesis "Molekular genetische Untersuchungen zur plasmidkodierten Arsenit und Arsenatrestistent bei Staphylococcen", by Dr. Ralf Rosenstein (available from the Technische Universitat, Munich, West Germany). The resulting plasmid was then digested with EcoR1.
b) Chromosomal DNA was isolated from *S. gallinarum* (DMS 4616) and was digested with EcoR1. A 4.7 kb fragment containing the gallidermin structural gene in a 2.4 kb long sequence between HindIII and EcoR1 restriction sites was isolated using as a primer the sequence. 5′ CAC ATC CAG GAG TAC 3′
c) The 4.7 kb Fragment was then ligated into the EcoR1 site of the digested pCUI plasmid from step a) to give a plasmid designated pCUgdm1.

### 2. Preparation of a S. epidermis host

In this example a mutant strain of *S. epidermis* DSM 3095 incapable of producing epidermin was isolated.

The mutagenesis was carried out on a strain which was characterized by chromosomally coded Rifampicin resistance (20 ug/ml).

*S. epidermis* DSM 3095 grown on Agar plates was used to inoculate 30 ml basic broth medium which was cultivated overnight. 0.5 ml of the overnight cultivation was then used to inoculate 50 ml of production medium which was shake cultivated at 37°C for three hours.

Cells were removed from the cultivation medium and suspended in 4.5 ml pre-warmed TM-Buffer (30 mM Tris-Maleate pH 6.5 (the resulting solution is designated Solution A)).

The solution A was checked for spontaneous mutations and for cell count (1.25 x 10¹⁰ cells/ml).

4 ml of solution A was thoroughly shaken with 1 ml ethyl methyl sulphonate (final concentration 47 µg/ml) and then maintained under shaking at 37°C for one hour.

Cells were then extracted from the cultivation broth, washed twice in TM-Buffer and resuspended in 5 ml TM-Buffer (the resulting solution was designated Solution B and contained mutated cells).

Solution B was found to contain 2 x 10⁸ cells/ml which corresponds to survival rate of 1.6%.

50 ml of solution B was added to 5 ml production medium and grown overnight at 37°C (phenotypic expression). The resulting solution was designated Solution C. A cell count showed 7.3 x 10⁸ cells/ml.

The solution was plated on BM-Agar plates and individual colonies were picked out. These were used to inoculate test plates (consisting of BM-Agar to which *Micrococcus luteus* has been laid on the surface). Those colonies which had no inhibitory effect on *M. luteus* were selected as non-producers of Epidermin.
BM Agar contains per liter:
10 gm Peptone No. 140
5 gm Yeast extract
1 mg Glucose
5 mg NaCl
1 mg K₂HPO₄
pH 7.5

A mutation rate of about 3% was noted.

The 45 non-producers which were found were sub-cloned 20 times to yield 16 stable non-producers.

All stable non-producers were found to contain the wild type plasmid pEpi32. From the restriction pattern this is identified as identical to the plasmid in the wild type strain.

### Transformation of non-producing S. epidermis

750 ml of BM-medium was inoculated with 5 ml of medium obtained by overnight cultivation of a stable non-producing strain, and the inoculated medium was shake cultivated in a 2 liter flask at 37°C with a shake speed of 120 rpm.

The initial optical density of the inoculated BM-medium was 0.03-0.04. When the optical density had reached 0.45-0.55 the cells were removed by centrifugation in a GS.-3-Rotor at 8500 rpm for 15 minutes at 4°C. The isolated cells were then washed successively in 750, 350, 40 and 10 ml of 10% glycerin, suspended in 2-3 ml 10% glycerin, and frozen in 110 ml portions in ERGs at -70°C. The cell count amounted to 1-5 x 10¹⁰/ml.

The frozen cells were thawed at room temperature for 5 minutes, then 50 µl of cell suspension was incubated in an ERG with 2 µl plasmid pCUgdm1 in TE-Buffer for 30 minutes at room temperature.

The mixture was then introduced into an electroporation cuvette having a 0.2 cm electrode gap and immediately electroporated. Thereafter the cells were rapidly resuspended in 950 µl SMMP50-medium, transferred into a 2.5 ml ERG and shaken for 90 minutes at 37°C. The ERGs were inclined at 45° in order to provide for a good aeration of the medium.

SMMP50-medium contains pro 100 ml, 55 ml 2SMM, 40 ml 4 PAB and 5 mol 5% BSA. The 2SMM contains 1 mol saccharose, 0.04 mol maleic acid, 0.04 mol MgCl₂ and NaOH to pH 6.5. 4 PAB is a solution of 7 g/100 ml of Gibco antibiotic medium 3.

The cell suspension is diluted and spread on a BM-Agar containing gallidermin which is incubated for 20 hours at 37°C.

Testing of growing strains which produce gallidermin was carried out by selection of colonies from a *M. luteus* test plate and by cultivating the respective selected colonies and determining the presence of gallidermin by HPLC.

Three pCUgdm1 transformed mutants capable of producing gallidermin were located.

### Determination of the presence of gallidermin produced by pCUgdm1 transformed S. epidermin

### a) Bio assay

FP-Agar was inoculated with *M. luteus* ATCC 9341 and incubated at 37°C for 18 hours. Half of the produced culture was removed with a loop and suspended in 100 ml FP-medium and was cultivated for 8 hours at 36°C. The cultivation was stopped when the optical density reached 1.0. FP-Agar was inoculated with 0.5% of this suspension, each 10 ml was poured into a Petri dish and stored for 3 weeks at 4°C.

The Plate diffusion test was carried out as described in Zähner and Maas, "Biology of Antibiotics", Springer Verlag, Berlin 1972. 10 ul of culture filtrate from cultivation of the transformed *S. epidermin* was captured on a filter paper and dried. The paper was placed on the test plate which was then incubated for 24 hours at 37°C.

### b) HPLC

The selected transformed strain was cultivated for 26 hours in the production medium. The culture broth was centrifuged for 10 minutes at 13.000 rpm.

The isolated culture liquid was then subject to HPLC on a SP 8.700 liquid chromatography apparatus (Spectra Physics, Darmstadt, FRG) using as the mobile phase A) H₂O with 0.5% 70% perchloric acid and B) Acetonitrile. Column packings were Nucleosil -100 C-18 of grain size 7 um and column sizes 125 mm x 4.6 mm I.D. and 20 mm x 4.6 mm ID for the pre-column.

Gradients were as follows:

| time (min.) | A [%] | B [%] |
|---|---|---|
| 0 | 77.5 | 22.5 |
| 8 | 63.0 | 37.0 |
| 8.5 | 0 | 100 |
| 9.5 | 0 | 100 |
| 10 | 77.5 | 22.5 |
| 14 | 77.5 | 22.5 |

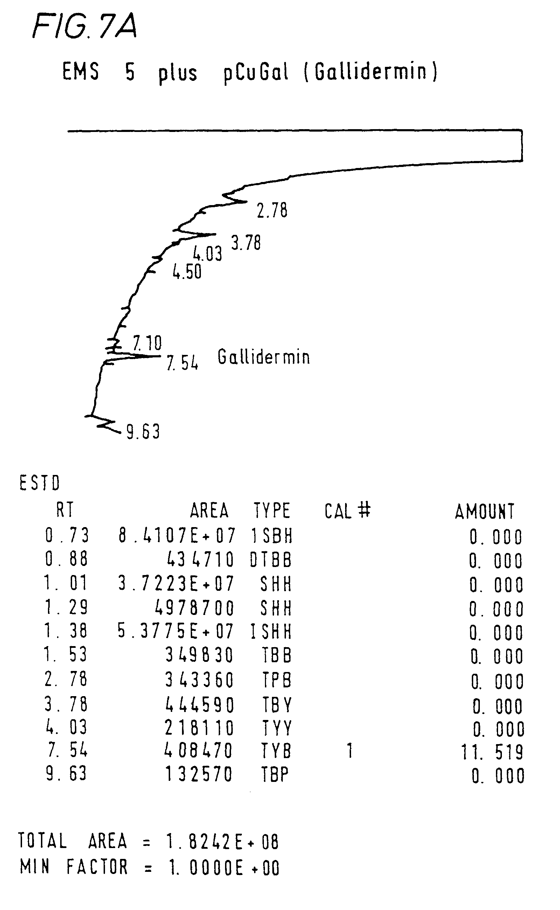

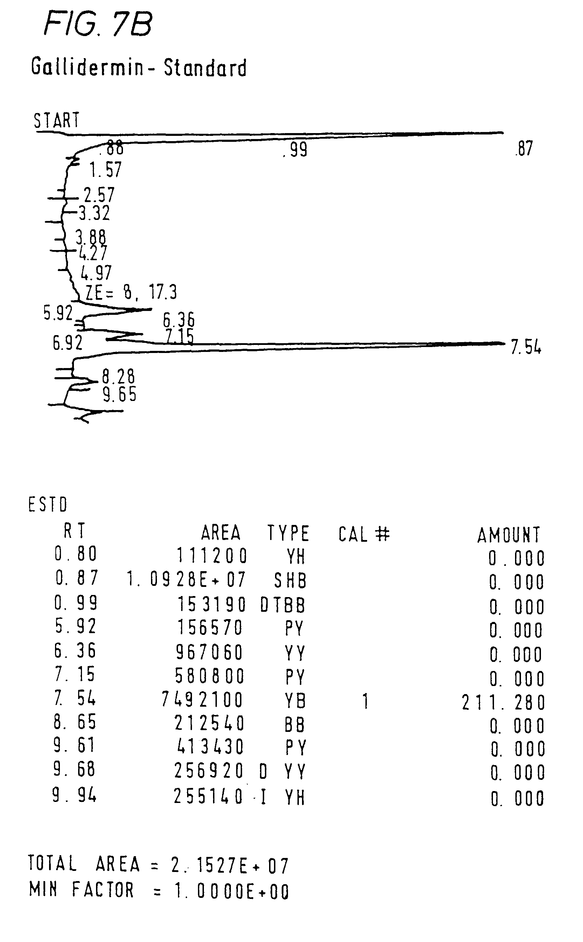

The resulting chromatogram is shown in Figure 7A. A standard curve is shown in Figure 7B showing that gallidermin elutes at 7.54 minutes.

The following were used as culture medium.

| 1. FP-Agar | |
|---|---|
| Meat extract | 4 g |
| Peptone | 10 g |
| NaCl | 3 g |
| Na₂HPO₄ | 5 g |
| Glucose | 10 g |
| Complex agar | 15 g |
| Water | 1 liter |
| pH | 7.2 |

| 2. FP-Medium | |
|---|---|
| Meat extract | 4 g |
| Peptone | 10 g |
| NaCl | 3 g |
| Na₂HPO₄ | 5 g |
| Glucose | 10 g |
| Water | 1 liter |
| pH | 7.2 |

| 3. Production medium | |
|---|---|
| Meat extract | 33 g |
| Malt extract | 30 g |
| NaCl | 40 g |
| Calcium Hydroxide | 3.8 g |
| Water | 1 liter |
| pH | 6.5 |

### Example 3

### Plasmid Isolation

Plasmid DNA from *S. epidermis* Tü3298 was isolated according to a modified procedure of Norick *et al.,Ann. NY-Acad. Sci. 182*:279-294 (1971). *S. epidermis* was grown on BM-media (1% peptone 140, Gibco, Neu-Isenburg, F.R.G., 0.5% yeast extract, Difco, Detroit, USA, 0.1% glucose, 0.5% NaCl and 0.1% K₂HPO₄ x 2H₂O) until stationary phase. Cells were centrifuged and washed twice with 0.5 M EDTA. The pellet was resuspended in 80 ml NaCl buffer (50 mM Tris/HCl, pH 7, 50 mM EDTA, 2.5 M NaCl), 1.5 ml lysostaphin solution (0.5 mg/ml, Sigma, Heidelberg, F.R.G.) was added and the suspension was incubated at 37°C for 20 min. Cells were lysed by the addition of 80 ml lysis buffer (50 mM Tris/HCl, pH 8, 300 mM EDTA, 500 mM Brij., 40 mM sodium deoxycholate and kept on ice for 1 h. The lysate was centrifuged (30 min, 13,000 rpm, 4°C) and the supernatant was mixed with one quarter of its volume with 50% solution of PEG-6000. Plasmid DNA was precipitated at 4°C overnight. The DNA suspension was centrifuged (20 min, 13,000 rpm, 4°C), resuspended in 8 ml TE buffer and 50 µl of proteinase K solution (20 mg/ml) was added. After incubation at 37°C for 15 min the DNA was precipitated with ethanol and further purified by CsCl centrifugation (1 g CsCl/ml, 40,000 rpm, 40 h, 20°C).

### RNA isolation and electrophoreses

*S. epidermidis* was grown on SMS minimum medium (Terzaghi *et al.*, *Appl. Microbiol. 29*:807-813 (1975)) and RNA isolated therefrom, using a modified procedure similar to that described for *Bacillus subtilis* RNA (Ulmanen *et al., J. Bacteriol.* 162:176-182 (1985)). Cells were lysed with lysostaphin (0.1 mg/ml) in protoplasting buffer and incubation was performed at 37°C. Total RNA was glyoxylated (McMaster *et al.*, *Proc. Natl. Acad.* Sci. USA 74:4835-4839 (1977)) and separated on a 1.2% agarose gel using 10 mM Na₂PO₄, pH 7, as electrophoresis buffer. RNA was stained with ethidium bromide and blotted to a nitrocellulose membrane (Scheider and Schuell, Dassel, F.R.G.) by capillary transfer with 20xSSC buffer (0.15 M NaCl, 0.015 M tri sodium citrate, pH 9). 23SrRNA and 16SrRNA were used as size standards.

### In vitro transcription

Single stranded RNA probes were obtained by cloning the respective fragment in a pSPT18/l9 vector system (Boehringer Mannheim, Mannheim, F.R.G.). The plasmids were linearized with EcoRI or HindIII to get a linear DNA template. For transcription the protocol in Melton *et al*., *Nucl. Acid Res. 12*:7035-7056 (1984), was modified according to the instructions of the commercial supplier. T7-RNA polymerase or SP6-RNA polymerase was used in the presence of α³²P-CTP (800 Ci/mMol). Unincorporated ribonucleotides were separated from labeled RNA by Sephadex G50 chromatography.

### Northern hybridization

RNA was transferred after electrophoresis according to Thomas, P.S., *Proc. Natl. Acad. Sci. USA 77*: 5201-5205 (1980). After 2 h incubation at 80°C the filter was shortly incubated in 20 Tris/HCl, pH 8, at l00°C to reverse glyoxylation. Afterwards filters were prehybridized at 42°C in 50% formamide, 5xSSC (0.15 M NaCl, 0.015 M tri sodium citrate, pH 9), 50 NaP0₄, pH 6.5, 0.1% ficoll 400 (Pharmazia, Freiburg, F.R.G.), 0.1% polyvinylpyrollidone, 0.1% bovine serum albumin and 0.25 mg/ml denatured salmon sperm DNA for 2h. After probe addition hybridization was performed in the same buffer at 42°C for 12 h. Filters were washed once in lxSSC, 0.1% SDS at 42°C for 15 min and exposed to Kodak-X Omat films at -70°C for 4 h. Thereafter filters were washed twice with 0.5 SSC, 0.1% SDS at 70°C for 15 min and autoradiograms were exposed at -70°C for 16 h. Next day washing was continued with 0.lxSSC, 0.1% SDS at 70°C for 30-60 min and afterwards again exposed to Kodak-X Omat films at -70°C for 3 days.

### Southern hybridization

For southern hybridization (Southern, E.M., *J. Mol. Biol. 98*:503-517 (1975)) 5' labeled oligonucleotides were used as probes at 23°C. Oligonucleotides were labeled with gamma³²P-ATP using 4T polynucleotide kinase (Boehringer Mannheim, Mannheim, F.R.G.). Oligonucleotides and primers were synthesized on a 391 DNA synthesizer (Applied Biosystems, Weiterstadt, F.R.G.) and used without further purification.

### DNA sequencing

DNA was sequenced radioactively and non-radioactively by the chain termination method (Sanger *et al*., *Proc. Natl. Acad. Sci. USA 74*:5463-5467 (1977)) using T7-DNA polymerase (Pharmazia, Freiburg, F.R.G.). Radioactive plasmid sequencing was performed as described in Hattori *et al*., *Anal. Biochem. 152*:232-238(1984) with appropriate primers. The 3.6 kb BamHI/PstI fragment was sequenced non-radioactively on an Applied 373A DNA sequenator (Applied Biosystems, Weiterstadt, F.R.G.). The respective fragment was cloned in phagemid pBSK-/+. The construction was digested with BamHI and SacI and the linearized DNA was unibidirectionally digested from the 5' end with exonuclease III (Boehringer Mannheim, Mannheim, F.R.G.) to obtain a set of nested deletions which were treated with mung bean nuclease (Boehringer Mannheim, Mannheim, F.R.G.) to receive blunt ends. After electrophoresis (1% agarose gel) fragments of appropriate size were isolated from the gel, religated and transformed into *E. coli* strain XL-1 Blue. Single stranded DNA was isolated by using helper phage CSM13 and sequenced with Taq Polymerase (Promega, Freiburg, F.R.G.) according to the protocol of the commercial supplier.

### Plasmid Construction

The staphylococcal tetracycline resistance plasmid pT181 has been sequenced (Kahn *et aL, Plasmid 10*:251-259 (1983)) and found to contain a single NdeI site within the pre-gene which is not necessary for plasmid replication (Gennaro *et al.*, *J. Bacteriol*. *169*:2601-2610 (1987)). The multiple cloning site (mcs) of the *E. coli* vector pUCl9 (Yanisch-Perron *et al.*, *Gene 33*:103-119 (1985)) was inserted into the NdeI site to form pT18lmcs (see Figure 14).

A staphylococcus-*E. coli* shuttle vector, pCUI (Figure 10) was constructed from pCLP100, a derivative of the staphylococcal chloramphenicol resistance plasmid pC194 (Horinouchi *et al., J. Bacteriol. 150*:815-825 (1982)) and the E. *coli* vector pUCl9. PCUI is stably maintained in both hosts with an insert size up to approximately 6 kb. pT18lmcs and pCUI are compatible in staphylococci and were used to subclone DNA fragments from pTü32.

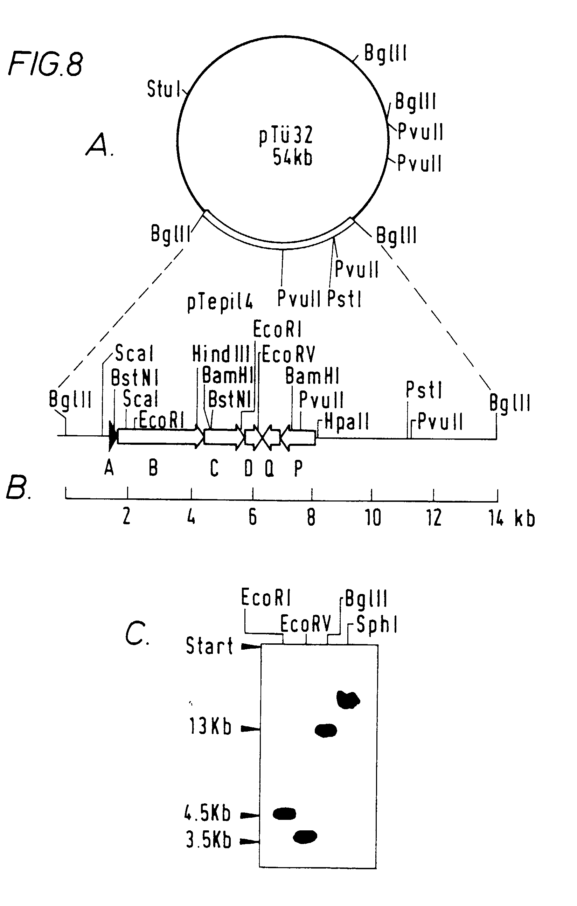

A HindIII fragment of pTü32 was cloned in pUCl9 and used as a probe in Southern hybridization to identify further restriction sites near the HindIII fragment (Figure 8C).

The 13.5 kbp BglII fragment of the 54 kbp episomal element pTü32 from *S. epidermidis* was subcloned in pT18lmcs to yield pTepil4 (Figure 8A). For DNA sequencing subclones were made in the *E. coli* vector pUCl9 (Yanisch-Perron *et al.*, *Gene* 33:103-119 (1985)) and pBluescript II^{R} (Stratagene, Heidelberg, F.R.G). Single stranded RNA probes were obtained from DNA cloned in vector pSPTl8/l9 (Boehringer Mannheim, Mannheim, F.R.G.).

### Gene Analysis

Sequencing the DNA region adjacent to the epidermin structural gene, epi A, revealed five additional complete open reading frames epi B, C, D, P and Q inside the 13.5kbp BglII fragment of pTü32.

As can be seen in Figure 9, directly adjacent to the sequence encoding for EpiA separated by only 50 nucleotides from the epiA ochre codon there is a large open reading frame preceded by a S/D sequence which spans 2,970 bp. A TTG codon for leucine which can also act as a translation start codon in staphylococci is in appropriate distance (86bp) to a S/D sequence. This open reading frame is designated epiB and as described herein can successfully be used for the complementation of epidermin biosynthesis mutants and an essential role in epidermin biosynthesis.

The protein coded for by epiB, starting from the TTG (Leu) has a molecular weight of about ll5kDa, a net charge of -3 at pH7, and is moderately hydrophobic (41% hydrophobic residues) as may also. be predicted from a hydrophilicity plot according to Kyte *et al., J. Mol. Biol. 157*:105-132 (1982).

At the 3' end of epiB no palindromine structure characteristic of transcription termination can be seen. There is, however, a 122bp. overlap with an other reading frame epiC, shifted by -1 base pair also to be seen in Figure 9.

We have established this to be no artefact by independently cloning and sequencing the respective 47 kbp HindIII-fragment twice from two independent plasmid isolations. This was also confirmed by mutant complementation with an epiC containing fragment as described herein.

Inside the overlapping region of epiB and epiC reading frames the first TTG codon (Leu) which is only 36bp 3' to an AGGA element serves as a translational start codon, indicating that both reading frames overlap by about 40 codons. The actual amino-terminus of the EpiC protein was determined by N-terminal sequencing. Reading frame epiC encodes a protein with 445 amino acid residues commencing with starting codon TTG (Leu). The reading frame epiD directly follows 3' to epiC with a start ATG 86p 3' to a AGGAGG S/D sequence. 3' to epiD is a classical rho dependent transcription terminator structure; epiD encodes a protein of 181 amino acid residues with ATG (Met) on start codon.

None of the proteins EpiB, C, D, P and Q show any similarity with protein sequences filed in the protein data bases Swiss Prot and Gene Bank, and thus represent unknown types of enzymes and regulatory proteins.

### Transcription of the biosynthetic genes

Single stranded RNA probes were obtained by cloning the desired fragment in a pSPT 18/19 vector system (Boehringer Mannheim, Mannheim, F.R.G.) as described above.

Two transcripts differing considerably in size were obtained as illustrated in Figure 10. A hybridization probe specific of epiA identified a small transcript of about 300bp. Transcripts of similar size were also found for the lantibiotics nisin (Buchmann *et al*., *J. Biol. Chem. 263*:16260-16266 (1988)) and subtilin (Banerjee *et al*., *J. Biol. Chem. 263*:9508-9514 (1988)). Additionally a large transcript of approximately 5kb can be identified with a hybridization probe specific for epiB. As there were no *E. coli*-like promoter sequences in front of epiB, whereas appropriate sequences were located 5' to epiA it can be seen that the epiA promoter acts as a promoter for a polycistronic mRNA.

### Downstream open reading frames

The open reading frames epiP and epiQ are located on the opposite DNA to epiB, C and D with epiQ sharing a termination structure with epiD a perfect hairpin with a 6bp loop.

Exactly within this loop structure the TAA stop codons for both reading frames epiD and epiQ share two of three nucleotides.

The epiP reading frame starts with an ATG codon which is in appropriate distance (6 bp) to a S/D sequence. Taking the ATG codon as the translational start of epiP a protein of 461 amino acid residues with molecular weight of 51.8kD. epiP shares characteristic homologies with the conserved amino acid motives of serine proteases (see Figure 11) indicating that epiP is implicated in cleaving the mature lantibiotic from the modified prepeptide.

The epiQ reading frame also starts with an ATG codon and encodes 205 amino acid residues (Figure 9). A S/D sequence is present 6 bp distance to the ATG codon and a molecular weight of 243 kD can be deduced from the DNA sequence. The epiQ protein shares characteristic homologies with PhoB (see Figure 12) which is a positive regulatory factor for the phosphate regulatory of *E. coli* so that epiQ is implicated as a regulatory factor in lantibiotic synthesis.

Preceding epiP is an *E. coli*-like -10 region (5'-TATAAA) 12bp in front of the S/D sequence which may serve as a promoter in staphylococci. The distance between the epiP stop codon and the ATG start codon of epiQ is only 10 nucleotides and the epiQ S/D sequence overlaps with the epiP termination codon as shown in Figure 9.

5' to epiA, B, C, D a further reading frame with opposite orientation can be seen which potentially encodes a maximum of 148 amino acids. A characteristic S/D sequence is present but none of the previously described start codons for staphylococci (ATG, TTG, GTG). With a -1 frame shift a further reading frame follows which exceeds the isolated BglII fragment illustrated in Figure 9.

These two reading frames are homologous to a single open reading frame, gdmY, identified adjacent to the structural gene of gallidermin (Schnell, N., *Biosynthese der Peptid*-*Antibiotika Epidermin und Gallidermin;* Doctoral Thesis, University of Tubingen, F.R.G. (1989)). The homologous reading frames on the *S. epidermis* plasmid are designated epiY' and epiY".

### Example 4

*S. carnosus* TM300 was transformed with the plasmid pTepil4, prepared as described above, using standard techniques. The transformed strain was then grown on BM-media (see above).

The resulting transformants were found to be capable of inhibiting the epidermin sensitive tester strain *Micrococcus luteus* ATCC9341. In this assay 1 ml of an overnight culture of *M. luteus* (adjusted to an OD₅₇₈ of 1.0) was added to 500 ml molten BM-Agar. Petri dishes usually contained 10 ml of this agar. Dilutions of *S. epidermidis* cultures were spread on the agar surface. Epidermin positive colonies were detected as a zero of growth inhibition of *M. luteus* around the colonies.

Cells were grown on 3% meat extract, 3.8% malt extract, 0.6% CaCl₂x2H₂0 and 4.6% NaCl, pH6.5. According to the transformation used, tetracycline or chloramphenicol was added. After 24 h incubation (37°C, 160 rpm) in 500 ml Erlenmeyer flasks with one extension containing 100 ml medium, the culture both was centrifuged at 10,000 rpm in a Servall centrifuge for 10 min.

Supernatants of liquid transformant cultures were purified by adsorption chromatography (XAD1180, impurities eluted with water/methanol (1:1) and epidermin eluted with methanol/0.1N HCI (9:1); after evaporation the eluate was adjusted with 3N NaOH to pH 3.5 and filled up with water to 10 ml) and detected by HPLC chromatography. The inhibitory activity co-migrated with mature epidermin at 6.75/6.76 min (see Figures 13A and 13B). Untransformed *S. carnosus* culture media treated similarly had no peak in this elution region (6.72 to 6.79 min, Figure 13C). These results clearly confirmed the heterologous epidermin biosynthesis in *S. carnosus* and demonstrated that pTepil4 contains all information necessary for epidermin biosynthesis.

As pTepil4 contains the 13.5kbp BglII fragment this indicates that the epiY' and epiY" reading frames are not necessary for the production of epidermin in this system as epiY' lacks a translational start codon and epiY" is incomplete on this fragment.

### Example 5

A number of epimutants of *S. epidermidis* Tü3298 were prepared by ethylmethane sulfonate (EMS) mutagenesis. This procedure was carried out according to Miller, J.H., *Experiments in molecular genetics,* Cold Spring Harbor Laboratory; Cold Spring Harbor, N.Y. (1972). The mutants were screened for epidermin production, or lack of epidermin production using the *M. luteus* assay described above. Epimutants were transferred several times to test their stability. Of the 40 epimutants isolated, only 10 were stable; the unstable mutants produced epidermin again after several transfers. All stable epimutants still contained plasmid pTü32 which suffered no deletions or rearrangements as tested by restriction endonuclease analysis. The 10 epimutants were used for complementation studies.

Various restriction fragments of plasmid pTü32 were cloned in *S. carnosus* to test for heterologous epidermin production. The fragments were inserted into plasmid vectors T181mcs and pCUI as described above and the various ORFs which were subcloned as shown in Figure 16B.

Cloning was first carried out in *S. carnosus* (by protoplast transformation (Götz *et al., FEMS Microbiol. Lett. 40*:285-288 (1987)) or *E. coli* (using CaCl₂; Cohen *et al.*, *Proc. Nat. Acad Sci. USA 69*:2110-2114 (1972)) and then the recombinant plasmids were isolated and transferred into the various *S. epidermidis* epi mutants by electroporation (Augustin *et al.*, *FEMS Microbiol. Lett. 66*:203-208 (1990)). Enzymes used for molecular cloning were obtained from Boehringer Mannheim (Mannheim, F.R.G.), BRL (Eggenstein, F.R.G.) or Pharmacia (Sweden). This indirect transformation method was necessary since transformation of *S. epidermin* strains was only successful with circular covalently closed (ccc) plasmids; when ligation products were used, transformants could only be isolated occasionally.

The results of the complementation studies are summarized in Table 1.

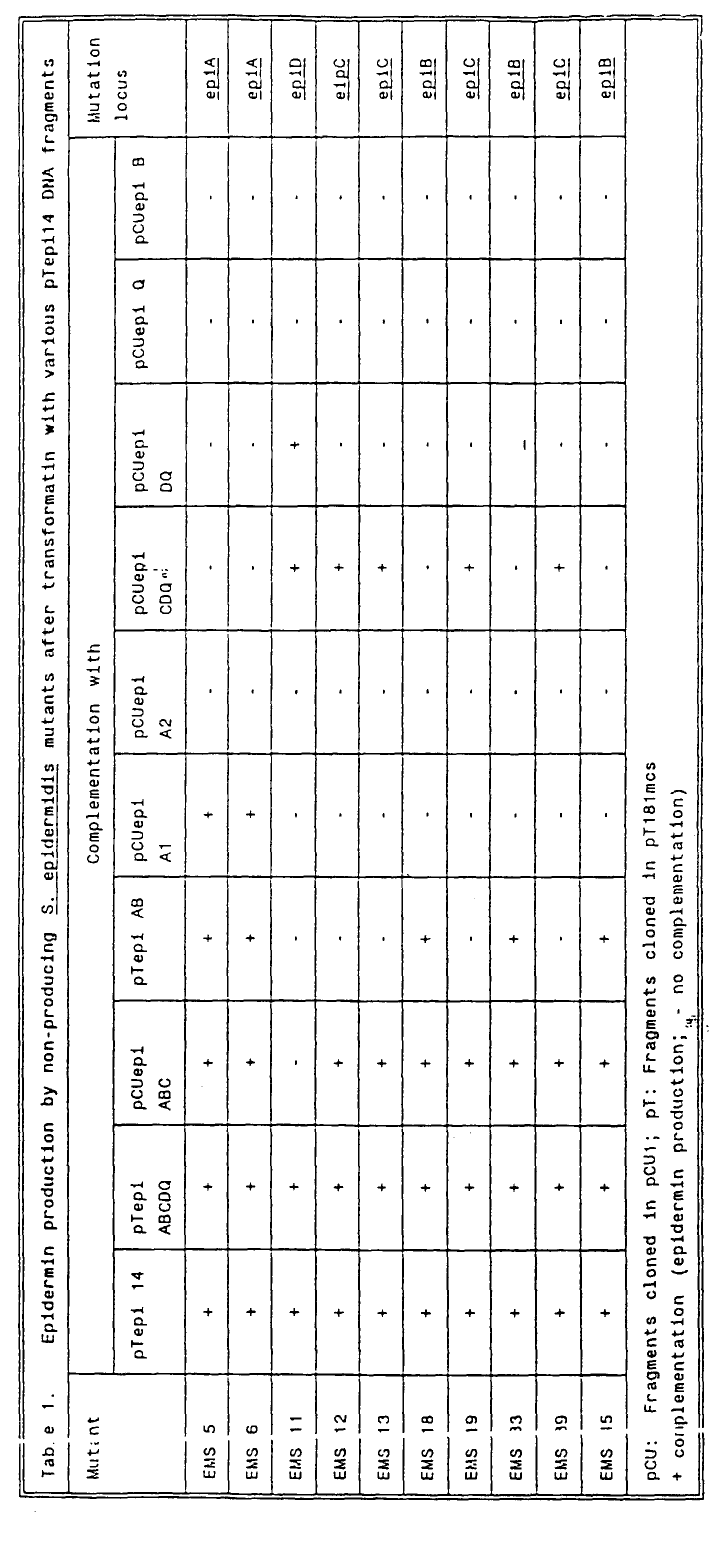

A series of plasmids were constructed which carry various epi genes (A, B, C, D, P and Q) (Figure 16B). Two plasmids pTepil4 and pTepiABCDQ were able to complement all epimutants. The other constructed plasmids pCUepiABC, pTepiAB, pCUepiCDQ, pCUepiB, pCUepiA₁, pCUepiA₂, pCUepiDQ and pCUepiQ contained the indicated genes.

The various plasmids were able to complement only certain classes of mutants which are classified herein as follows:
EMS 5 and 6 - epiA mutants,
EMS 18, 33 and 45 - epiB mutants,
EMS 12, 13, 19 and 39 - epiC mutants,
EMS 11 - epiD mutant.

The results as shown below indicate at least that the four ORFs epiA, B, C and D are required for epidermin biosynthesis.

The plasmid pCUepiA₁ carries the structural gene epiA as the only complete ORF and an additional 1400bp upstream and 602bp downstream, the latter encoding 190 amino acids of the epiB N-terminus. Transformation using pCUepiA₁ resulted in the complementation of the epidermin mutants EMS 5 and 6 identifying them as epiA mutants. The smaller epiA-containing Scal fragment cloned in both orientations in pCUepiA₂ failed to complement the epimutants as the epiA promoter was cut by this enzyme.

pCUepiB carries a BstN1 fragment containing the complete epiB and an upstream region of 100bp which includes 75bp of the 3' terminus of epiA; the epiA promoter is missing. Transformation with pCUepiB failed to complement any *S. epidermis* mutant to epidermin production, indicating that epiB lacks its own promoter and is very likely co-transcribed from the epiA promoter.

This is in agreement with the results obtained with pTepiAB (Figure 16B; Table 1) which contains epiA promoter and the complete epiA and B genes and the use of which complements both the epiA and epiB mutants.

Plasmid pCUepiCDQ was able to complement both epiC and epiD mutants and plasmid pCUepiDQ was only able to complement the epiD mutant (Table 1). The complementation was independent of the orientation of the cloned DNA fragment. These results show that both epiC and epiD possess their own promoters.

### Example 6

The epiA mutated pTü32 derivatives were isolated from EMS 5 and 6 and the respective epiA ORFs were sequenced. Both plasmids had point mutations within epiA; in the EMS 5 plasmid the codon AGT (Ser³) was changed to AAT (Asn³) and in the EMS 6 plasmid the codon GGA (Gly¹⁰) was changed to GAA (Gln¹⁰); both these mutations were located at crucial sites within the unmodified epidermin.

### Example 7

An epiB (on a BstN1-fragment) was put under the control of the promoter on plasmid pPS4 (Figure 17). The resulting plasmid pPS4epiB was able to complement the epiB mutants EMS 18, 33 and 45. A plasmid containing epiB in the opposite orientation did not complement the mutations. This also establishes that pCUepiB was unable to complement any of the EMS mutants, because the epiA promoter is missing.

### Example 8

As described above, the presence of pTepi14 (Figure 16A) resulted in epidermin biosynthesis in *S. carnosus*; however, the presence of pTepiABCDQ did not. The minimum size of DNA required which leads to heterologous epidermin expression in *S. carnosus* was determined by complementing *S. carnosus* (pTepiABCDQ) with distally located DNA fragments (Figure 18). Transformation of *S. carnosus* (pTepiABCDQ) with plasmids pCA44-90, pCA44-91 and pCA44-92 led to epidermin production, pCA44-92 containing the complete epiQ and epiP ORFs consisted of the smallest DNA fragment able to complement epidermin production. These results indicate that the epidermin biosynthetic genes are clustered within an 8kb DNA fragment containing the six ORFs; epiA, B, C, D, Q and P and that no other genes are involved in epidermin biosynthesis.

In these examples staphyloccal plasmid DNA was prepared by the cleaved lysate method (Makino *et al*., *J. Mol. Biol. 190*:37-44 (1986)). Cells were lysed by the addition of lysostaphin (8 µg/ml) and the DNA was isolated by CsCl-centrifugation. *E. coli* supercoiled plasmid DNA was prepared by the modified alkaline lysis method (Birnboim *et al*., *Nucl*. *Acid Res. 7*:1513-1518 (1979)).

The DNA sequence of the PCR-amplified epiA-containing fragment and the two mutated epiA regions of the *S. epidermis* mutants, EMS 5 and 6, was determined by double-stranded DNA sequencing using the dideoxy procedure (McMaster *et al*., *Proc. Natl. Acad. Sci. USA 74*:4835-4839 (1977)), the "sequence" list of Pharmacia and (α-³⁵S)-dATP from Amersham. Primers used for DNA sequencing and PCR amplification were synthesized using the DNA-synthesizer of Applied Biosystems. The sequences of the two primers for PCR amplification of epiA are as follows:

a)    5'-GGGTTTTAGG(TA)ATCCTTTTTAATAAATTTTTAGGAG-3'

b)    5′-CCTCAAAATTAAGACG(A)GAT(G)CCTCTATTGAAGCCC-3′

Primer a) binds in front of the RBS of epiA and primer b) after the epiA stop codon. These bases indicated by bold letters represent (shown in brackets) used to create BamHI sites in front and at the end of epiA; the epiA promoter is absent in the amplified DNA fragment.

For determination of the DNA sequence of the mutated epiA in the mutants EMS 5 and 6, plasmid pTü32 was isolated and the DNA region was amplified by PCR using another set of DNA primers binding upstream of the postulated epiA promoter region (5'-GGTTTGGTTATTTTCC-3') and downstream of the stop codon (5'-CCTCAAAATTAAGACAGAGCCTC-3'); the DNA sequence of epiA is also shown in Schnell *et al*., *Nature (Lond.) 333*:276-278 (1988).

### Example 9

The epi D gene was isolated from the plasmid pTepi 14, multiplied by PCR amplification and cloned into the StuI-restriction site of vector pIH902 (New England, Biolabs) by "blunt end" ligation, with the result that the epi D gene is fused without any intervening base pairs immediately at the Factor Xa-cleavage site of vector pIH902, which was then transformed into *E. coli*.

Cultivation of the *E. coli* resulted in expression of the enzyme Epi D fused to the Maltose binding protein of *E. coli*. The resulting fusion protein was purified by affinity chromatography on Amylose column material.

It was found that the enzyme epiD could be cleaved from the fusion protein in low yield by means of Factor Xa. A modification of the amino acid sequence at the cleavage region will enable the cleavage rate to be improved.

The fusion protein was sequenced at the DNA level from the fusion position to the 3' end of epiD. The epiD sequence corresponded to the wild type sequence of *S. epidermis*.

Plasmid pCA44 is deposited in Staphylococcus carnosus TM 300 at the DSM Deutsche Sammlung von Mikroorganismen und Zellkulturen GmbH, Mascheroder Weg 1b, D-3300 Braunschweig, Germany, under No. 6863, the deposit being dated 23rd December 1991.

Plasmid pPS4 is deposited in Stephylococcus carnosus TM 300 under No. 6864 at the same depositary, deposit being dated 23rd December 1991.

## Claims (Claims for the following Contracting State(s): AT, BE, CH, DE, DK, FR, GB, IE, IT, LI, LU, NL, PT, SE)

1. A recombinant DNA molecule, characterised in that it is capable of hybridising under stringent conditions to an Epi B, or Epi C, or Epi D, or Epi P, or Epi Q protein encoding nucleotide molecule having a sequence as set forth in Figure 9 comprised in the BglII fragment of the 54 kbp episomal element of pTü32 from S. epidermidis, said micro-organism being deposited at the Deutsche Sammlung für Mikroorganismen under deposition No. DSM 3095, and said DNA molecule coding for a protein having Epi B, or Epi C, or Epi D, or Epi P, or Epi Q enzymatic activity.

2. A recombinant DNA molecule according to claim 1, characterised in that it encodes at least one of the proteins Epi B, Epi C, Epi D, Epi P, or Epi Q having an amino acid sequence as set forth in Figure 9.

3. A recombinant DNA molecule according to claim 1 or 2, characterised in that it comprises at least one of the nucleic acid sequences as set forth in Figure 9 coding for the proteins Epi B, Epi C, Epi D, Epi P or Epi Q.

4. A plasmid, characterised in that it comprises a DNA molecule according to any one of claims 1 to 3, said DNA molecule being under the control of a promoter regulator sequence and coding for a protein having Epi B, Epi C, Epi D, Epi P, or Epi Q activity but wherein one or more of the DNA sequences coding for the other said proteins or Epi A are absent.

5. A protein, characterised in that it is encoded by a DNA molecule according to any one of claims 1 to 4.

6. A method of producing a protein according to claim 5, characterised in that a DNA molecule as defined in any one of claims 1 to 4 is inserted into a plasmid vector such that said DNA molecule is under the control of a promoter regulator, inserting the resulting plasmid vector into a suitable host, culturing said host so that the protein in expressed, and isolating the protein.

7. A method of producing a protein having at least one of the structural features of a lantibiotic, characterised in that a DNA sequence coding for a pre-determined amino acid sequence is inserted in a vector plasmid such that it is under the control of a regulator promoter, transforming the resulting plasmid vector into a suitable host and cultivating the host so as to express the said DNA and isolating the protein, wherein the host is also transformed by a DNA sequence or sequences as defined in any one of claims 1 to 4 which is expressed during the said cultivation ofthe host.

8. A method according to claim 7, characterised in that the DNA sequence coding for the pre-determined amino acid sequence and the DNA sequence as defined in any one of claims 1 to 3 is inserted in the same plasmid vector which is used to transform the host.

9. A method according to claim 7 or 8, characterised in that said DNA sequence contains or is inserted immediately downstream of a DNA sequence coding for the pre-peptide sequence -30 to -1 of pre-epidermin.

10. A fusion protein, characterised in that it comprises a protein according to claim 5 and an auxiliary protein.

11. A fusion protein, characterised in that it comprises a protein according to claim 5 fused to an auxiliary protein such that the junction between the protein according to claim 5 and the auxiliary protein can be cleaved by an enzyme

12. A fusion protein according to claim 10, characterised in that said auxiliary protein facilitates secretion of the fusion protein from a selected host

13. A fusion protein according to claim 10, characterised in that the auxiliary protein facilitates purification by affinity chromatography.

14. A fusion protein according to claim 10, characterised in that the auxiliary protein is the Maltose binding protein from E. coli.

15. A recombinant DNA molecule, characterised in that it is coding for a fusion protein according to claim 10.

## Claims (Claims for the following Contracting State(s): ES, GR)

1. A recombinant DNA molecule, characterised in that it is capable of hybridising under stringent conditions to an Epi B, or Epi C, or Epi D, or Epi P, or Epi Q protein encoding nucleotide molecule having a sequence as set forth in Figure 9 comprised in the BglII fragment of the 54 kbp episomal element of pTü32 from S. epidermidis, said micro-organism being deposited at the Deutsche Sammlung für Mikroorganismen under deposition No. DSM 3095, and said DNA molecule coding for a protein having Epi B, or Epi C, or Epi D, or Epi P, or Epi Q enzymatic activity.

2. A recombinant DNA molecule according to claim 1, characterised in that it encodes at least one of the proteins Epi B, Epi C, Epi D, Epi P, or Epi Q having an amino acid sequence as set forth in Figure 9.

3. A recombinant DNA molecule according to claim 1 or 2, characterised in that it comprises at least one of the nucleic acid sequences as set forth in Figure 9 coding for the proteins Epi B, Epi C, Epi D, Epi P or Epi Q.

4. A plasmid, characterised in that it comprises a DNA molecule according to any one of claims 1 to 3, said DNA molecule being under the control of a promoter regulator sequence and coding for a protein having Epi B, Epi C, Epi D, Epi P, or Epi Q activity but wherein one or more of the DNA sequences coding for the other said proteins or Epi A are absent.

5. A protein, characterised in that it is encoded by a DNA molecule according to any one of claims 1 to 4.

6. A method of producing a protein according to claim 5, characterised in that a DNA molecule as defined in any one of claims 1 to 4 is inserted into a plasmid vector such that said DNA molecule is under the control of a promoter regulator, inserting the resulting plasmid vector into a suitable host, culturing said host so that the protein in expressed, and isolating the protein.

7. A method of producing a protein having at least one of the structural features of a lantibiotic, characterised in that a DNA sequence coding for a pre-determined amino acid sequence is inserted in a vector plasmid such that it is under the control of a regulator promoter, transforming the resulting plasmid vector into a suitable host and cultivating the host so as to express the said DNA and isolating the protein, wherein the host is also transformed by a DNA sequence or sequences as defined in any one of claims 1 to 4 which is expressed during the said cultivation of the host.

8. A method according to claim 7, characterised in that the DNA sequence coding for the pre-determined amino acid sequence and the DNA sequence as defined in any one of claims 1 to 3 is inserted in the same plasmid vector which is used to transform the host.

9. A method according to claim 7 or 8, characterised in that said DNA sequence contains or is inserted immediately downstream of a DNA sequence coding for the pre-peptide sequence -30 to -1 of pre-epidermin.

10. A method of producing a fusion protein, characterised in that a protein according to claim 5 is fused to an auxiliary protein

11. A method of producing a fusion protein, characterised in that a protein according to claim 5 is fused to an auxiliary protein and an enzymatically cleavable sequence is introduced at the junction between the protein according to claim 5 and the auxiliary protein

12. A method according to claim 10, characterised in that said auxiliary protein facilitates secretion of the fusion protein from a selected host.

13. A method according to claim 10, characterised in that the auxiliary protein facilitates purification by affinity chromatography.

14. A method according to claim 10, characterised in that the auxiliary protein is the Maltose binding protein from E coli

## Patentansprüche (Patentansprüche für folgende(n) Vertragsstaat(en): AT, BE, CH, DE, DK, FR, GB, IE, IT, LI, LU, NL, PT, SE)

1. Rekombinantes DNA-Molekül, gekennzeichnet durch seine Fähigkeit zur Hybridisierung unter strikten Bedingungen zu einem für ein Protein Epi B oder Epi C oder Epi D oder Epi P oder Epi Q codierendes Nukleotid-Molekül mit einer Sequenz wie in Figur 9 angegeben, die im BglII-Fragment des 54 kbp episomalen Elementes von pTü32 aus S. epidermidis enthalten ist, wobei der genannte Mikroorganismus bei der Deutschen Sammlung für Mikroorganismen unter der Hinterlegungs-Nr. DSM 3095 hinterlegt ist und das genannte DNA-Molekül für ein Protein codiert, das eine enzymatische Aktivität von Epi B oder Epi C oder Epi D oder Epi P oder Epi Q aufweist.

2. Rekombinantes DNA-Molekül nach Anspruch 1, dadurch gekennzeichnet, dass es für mindestens eines der eine Aminosäure-Sequenz wie in Figur 9 angegeben enthaltenden Proteine Epi B, Epi C, Epi D, Epi P oder Epi Q codiert.

3. Rekombinantes DNA-Molekül nach Anspruch 1 oder 2, dadurch gekennzeichnet, dass es mindestens eine der für die Proteine Epi B, Epi C, Epi D, Epi P oder Epi Q codierenden Nucleinsäure-Sequenzen wie in Figur 9 angegeben umfasst.

4. Plasmid, dadurch gekennzeichnet, dass es ein DNA-Molekül nach einem der Ansprüche 1 bis 3 umfasst, wobei das genannte DNA-Molekül unter der Kontrolle einer Promotor-Regulator-Sequenz steht und für ein Protein codiert, das eine Aktivität von Epi B, Epi C, Epi D, Epi P oder Epi Q aufweist, bei welchem jedoch eine oder mehrere der für die anderen genannten Proteine oder für Epi A codierenden DNA-Sequenzen abwesend sind.

5. Protein, dadurch gekennzeichnet, dass es von einem DNA-Molekül nach einem der Ansprüche 1 bis 4 codiert wird.

6. Verfahren zur Herstellung eines Proteins nach Anspruch 5, dadurch gekennzeichnet, dass ein wie in einem der Ansprüche 1 bis 4 definiertes DNA-Molekül in einen Plasmid-Vektor eingeführt wird, derart, dass das genannte DNA-Molekül unter der Kontrolle eines Promotor-Regulators steht, der resultierende Plasmid-Vektor in einen geeigneten Wirt eingeführt wird, der gennante Wirt so kultiviert wird, dass das Protein exprimiert wird, und das Protein isoliert wird.

7. Verfahren zur Herstellung eines Proteins, das mindestens eine der strukturellen Eigenschaften eines Lantibiotikums aufweist, dadurch gekennzeichnet, dass eine für eine vorbestimmte Aminosäure-Sequenz codierende DNA-Sequenz in einen Plasmid-Vektor eingeführt wird, derart, dass sie unter der Kontrolle eines Promotor-Regulators steht, der resultierende Plasmid-Vektor in einen geeigneten Wirt transformiert wird und der Wirt so kultiviert wird, dass er die genannte DNA-Sequenz exprimiert, und das Protein isoliert wird, wobei der Wirt auch von einer wie in einem der Ansprüche 1 bis 4 definierten DNA-Sequenz bzw. -Sequenzen transformiert wird, die während der genannten Kultivierung des Wirtes exprimiert wird.

8. Verfahren nach Anspruch 7, dadurch gekennzeichnet, dass die für die vorbestimmte Aminosäure-Sequenz codierende DNA-Sequenz und die wie in einem der Ansprüche 1 bis 3 definierte DNA-Sequenz in dasselbe Plasmid eingeführt werden, das zur Transformation des Wirtes verwendet wird.

9. Verfahren nach Anspruch 7 oder 8, dadurch gekennzeichnet, dass die DNA-Sequenz eine für die Präpeptid-Sequenz -30 bis -1 von Präepidermin codierende DNA-Sequenz enthält oder unmittelbar flussabwärts davon eingebaut wird.

10. Fusionsprotein, dadurch gekennzeichnet, dass es ein Protein nach Anspruch 5 und ein Hilfsprotein umfasst.

11. Fusionsprotein, dadurch gekennzeichnet, dass es ein mit einem Hilfsprotein fusioniertes Protein nach Anspruch 5 umfasst, derart, dass die Verbindungsstelle zwischen dem Protein nach Anspruch 5 und dem Hilfsprotein von einem Enzym gespalten werden kann.

12. Fusionsprotein nach Anspruch 10, dadurch gekennzeichnet, dass das genannte Hilfsprotein eine Sekretion des Fusionsproteins aus einem ausgewählten Wirt erleichtert.

13. Fusionsprotein nach Anspruch 10, dadurch gekennzeichnet, dass das Hilfsprotein eine Reinigung durch Affinitäts-Chromatographie erleichtert.

14. Fusionsprotein nach Anspruch 10, dadurch gekennzeichnet, dass das Hilfsprotein das Maltose bindende Protein aus E. coli ist.

15. Rekombinantes DNA-Molekül, dadurch gekennzeichnet, dass es für ein Fusionsprotein nach Anspruch 10 codiert.

## Patentansprüche (Patentansprüche für folgende(n) Vertragsstaat(en): ES, GR)

1. Rekombinantes DNA-Molekül, gekennzeichnet durch seine Fähigkeit zur Hybridisierung unter strikten Bedingungen zu einem für ein Protein Epi B oder Epi C oder Epi D oder Epi P oder Epi Q codierendes Nukleotid-Molekül mit einer Sequenz wie in Figur 9 angegeben, die im BglII-Fragment des 54 kbp episomalen Elementes von pTü32 aus S. epidermidis enthalten ist, wobei der genannte Mikroorganismus bei der Deutschen Sammlung für Mikroorganismen unter der Hinterlegungs-Nr. DSM 3095 hinterlegt ist und das genannte DNA-Molekül für ein Protein codiert, das eine enzymatische Aktivität von Epi B oder Epi C oder Epi D oder Epi P oder Epi Q aufweist.

2. Rekombinantes DNA-Molekül nach Anspruch 1, dadurch gekennzeichnet, dass es für mindestens eines der eine Aminosäure-Sequenz wie in Figur 9 angegeben enthaltenden Proteine Epi B, Epi C, Epi D, Epi P oder Epi Q codiert.

3. Rekombinantes DNA-Molekül nach Anspruch 1 oder 2, dadurch gekennzeichnet, dass es mindestens eine der für die Proteine Epi B, Epi C, Epi D, Epi P oder Epi Q codierenden Nucleinsäure-Sequenzen wie in Figur 9 angegeben umfasst.

4. Plasmid, dadurch gekennzeichnet, dass es ein DNA-Molekül nach einem der Ansprüche 1 bis 3 umfasst, wobei das genannte DNA-Molekül unter der Kontrolle einer Promotor-Regulator-Sequenz steht und für ein Protein codiert, das eine Aktivität von Epi B, Epi C, Epi D, Epi P oder Epi Q aufweist, bei welchem jedoch eine oder mehrere der für die anderen genannten Proteine oder für Epi A codierenden DNA-Sequenzen abwesend sind.

5. Protein, dadurch gekennzeichnet, dass es von einem DNA-Molekül nach einem der Ansprüche 1 bis 4 codiert wird.

6. Verfahren zur Herstellung eines Proteins nach Anspruch 5, dadurch gekennzeichnet, dass ein wie in einem der Ansprüche 1 bis 4 definiertes DNA-Molekül in einen Plasmid-Vektor eingeführt wird, derart, dass das genannte DNA-Molekül unter der Kontrolle eines Promotor-Regulators steht, der resultierende Plasmid-Vektor in einen geeigneten Wirt eingeführt wird, der gennante Wirt so kultiviert wird, dass das Protein exprimiert wird, und das Protein isoliert wird.

7. Verfahren zur Herstellung eines Proteins, das mindestens eine der strukturellen Eigenschaften eines Lantibiotikums aufweist, dadurch gekennzeichnet, dass eine für eine vorbestimmte Aminosäure-Sequenz codierende DNA-Sequenz in einen Plasmid-Vektor eingeführt wird, derart, dass sie unter der Kontrolle eines Promotor-Regulators steht, der resultierende Plasmid-Vektor in einen geeigneten Wirt transformiert wird und der Wirt so kultiviert wird, dass er die genannte DNA-Sequenz exprimiert, und das Protein isoliert wird, wobei der Wirt auch von einer wie in einem der Ansprüche 1 bis 4 definierten DNA-Sequenz bzw. -Sequenzen transformiert wird, die während der genannten Kultivierung des Wirtes exprimiert wird.

8. Verfahren nach Anspruch 7, dadurch gekennzeichnet, dass die für die vorbestimmte Aminosäure-Sequenz codierende DNA-Sequenz und die wie in einem der Ansprüche 1 bis 3 definierte DNA-Sequenz in dasselbe Plasmid eingeführt werden, das zur Transformation des Wirtes verwendet wird.

9. Verfahren nach Anspruch 7 oder 8, dadurch gekennzeichnet, dass die DNA-Sequenz eine für die Präpeptid-Sequenz -30 bis -1 von Präepidermin codierende DNA-Sequenz enthält oder unmittelbar flussabwärts davon eingebaut wird.

10. Verfahren zur Herstellung eines Fusionsproteines, dadurch gekennzeichnet, dass ein Protein nach Anspruch 5 mit einem Hilfsprotein fusioniert wird.

11. Verfahren zur Herstellung eines Fusionsproteines, dadurch gekennzeichnet, dass ein Protein nach Anspruch 5 mit einem Hilfsprotein fusioniert wird und eine enzymatisch spaltbare Sequenz an der Verbindungsstelle zwischen dem Protein nach Anspruch 5 und dem Hilfsprotein eingeführt wird.

12. Verfahren nach Anspruch 10, dadurch gekennzeichnet, dass das genannte Hilfsprotein eine Sekretion des Fusionsproteins aus einem ausgewählten Wirt erleichtert.

13. Verfahren nach Anspruch 10, dadurch gekennzeichnet, dass das Hilfsprotein eine Reinigung durch Affinitäts-Chromatographie erleichtert.

14. Verfahren nach Anspruch 10, dadurch gekennzeichnet, dass das Hilfsprotein das Maltose bindende Protein aus E. coli ist.

## Revendications (Revendications pour l'(les) Etat(s) contractant(s) suivant(s): AT, BE, CH, DE, DK, FR, GB, IE, IT, LI, LU, NL, PT, SE)

1. Molécule d'ADN recombinée caractérisée en ce qu'elle est capable de s'hybrider dans des conditions strictes avec une molécule nucléotidique codant une protéine Epi B ou Epi C ou Epi D ou Epi P ou Epi Q ayant une séquence telle que représentée sur la figure 9 comprise dans le fragment BglII de l'élément épisomique de 54 kpb de pTü32 de S. epidermidis, ledit micro-organisme étant déposé auprès de la Deutsche Sammlung für Mikroorganismen sous le numéro de dépôt DSM 3095, et ladite molécule d'ADN codant une protéine ayant une activité enzymatique de Epi B ou Epi C ou Epi D ou Epi P ou Epi Q.

2. Molécule d'ADN recombinée selon la revendication 1 caractérisée en ce qu'elle code au moins l'une des protéines Epi B, Epi C, Epi D, Epi P ou Epi Q ayant une séquence d'acides aminés telle que représentée sur la figure 9.

3. Molécule d'ADN recombinée selon la revendication 1 ou 2 caractérisée en ce qu'elle comprend au moins l'une des séquences d'acide nucléique telles que représentées sur la figure 9 codant les protéines Epi B, Epi C, Epi D, Epi P ou Epi Q.

4. Plasmide caractérisé en ce qu'il comprend une molécule d'ADN selon l'une quelconque des revendications 1 à 3, ladite molécule d'ADN étant sous le contrôle d'une séquence promoteur-régulateur et codant une protéine ayant une activité de Epi B, Epi C, Epi D, Epi P ou Epi Q mais où une ou plusieurs des séquences d'ADN codant lesdites autres protéines ou Epi A sont absentes.

5. Protéine caractérisée en ce qu'elle est codée par une molécule d'ADN selon l'une quelconque des revendications 1 à 4.

6. Procédé de production d'une protéine selon la revendication 5 caractérisé en ce qu'une molécule d'ADN telle que définie dans l'une quelconque des revendications 1 à 4 est insérée dans un vecteur plasmidique de sorte que ladite molécule d'ADN est sous le contrôle d'un promoteur-régulateur, l'insertion du vecteur plasmidique résultant dans un hôte approprié, la culture dudit hôte de sorte que la protéine est exprimée et l'isolement de la protéine.

7. Procédé de production d'une protéine ayant au moins l'une des caractéristiques structurales d'un antibiotique, caractérisé en ce qu'une séquence d'ADN codant une séquence d'acides aminés prédéterminée est insérée dans un plasmide vecteur de telle sorte qu'elle est sous le contrôle d'un promoteur-régulateur, la transformation au moyen du vecteur plasmidique résultant d'un hôte approprié et la culture de l'hôte de manière à exprimer ledit ADN et l'isolement de la protéine, où l'hôte est aussi transformé par une séquence d'ADN ou des séquences d'ADN telles que définies dans l'une quelconque des revendications 1 à 4 qui sont exprimées pendant ladite culture de l'hôte.

8. Procédé selon la revendication 7 caractérisé en ce que la séquence d'ADN codant la séquence d'acides aminés prédéterminée et la séquence d'ADN telle que définie dans l'une quelconque des revendications 1 à 3 sont insérées dans le même vecteur plasmidique que celui qui est utilisé pour transformer l'hôte.

9. Procédé selon la revendication 7 ou 8 caractérisé en ce que ladite séquence d'ADN contient une séquence d'ADN codant la séquence de prépeptide -30 à -1 de la pré-épidermine ou est insérée immédiatement en aval de celle-ci.

10. Protéine de fusion caractérisée en ce qu'elle comprend une protéine selon la revendication 5 et une protéine auxiliaire.

11. Protéine de fusion caractérisée en ce qu'elle comprend une protéine selon la revendication 5 fusionnée à une protéine auxiliaire de sorte que la jonction entre la protéine selon la revendication 5 et la protéine auxiliaire peut être clivée par une enzyme.

12. Protéine de fusion selon la revendication 10 caractérisée en ce que ladite protéine auxiliaire facilite la sécrétion de la protéine de fusion à partir d'un hôte choisi.

13. Protéine de fusion selon la revendication 10 caractérisée en ce que la protéine auxiliaire facilite la purification par chromatographie d'affinité.

14. Protéine de fusion selon la revendication 10 caractérisée en ce que la protéine auxiliaire est la protéine de liaison au maltose provenant de E. coli.

15. Molécule d'ADN recombinée caractérisée en ce qu'elle code une protéine de fusion selon la revendication 10.

## Revendications (Revendications pour l'(les) Etat(s) contractant(s) suivant(s): ES, GR)

1. Molécule d'ADN recombinée caractérisée en ce qu'elle est capable de s'hybrider dans des conditions strictes avec une molécule nucléotidique codant une protéine Epi B ou Epi C ou Epi D ou Epi P ou Epi Q ayant une séquence telle que représentée sur la figure 9 comprise dans le fragment BglII de l'élément épisomique de 54 kpb de pTü32 de S. epidermidis, ledit micro-organisme étant déposé auprès de la Deutsche Sammlung für Mikroorganismen sous le numéro de dépôt DSM 3095, et ladite molécule d'ADN codant une protéine ayant une activité enzymatique de Epi B ou Epi C ou Epi D ou Epi P ou Epi Q.

2. Molécule d'ADN recombinée selon la revendication 1 caractérisée en ce qu'elle code au moins l'une des protéines Epi B, Epi C, Epi D, Epi P ou Epi Q ayant une séquence d'acides aminés telle que représentée sur la figure 9.

3. Molécule d'ADN recombinée selon la revendication 1 ou 2 caractérisée en ce qu'elle comprend au moins l'une des séquences d'acide nucléique telles que représentées sur la figure 9 codant les protéines Epi B, Epi C, Epi D, Epi P ou Epi Q.

4. Plasmide caractérisé en ce qu'il comprend une molécule d'ADN selon l'une quelconque des revendications 1 à 3, ladite molécule d'ADN étant sous le contrôle d'une séquence promoteur-régulateur et codant une protéine ayant une activité de Epi B, Epi C, Epi D, Epi P ou Epi Q mais où une ou plusieurs des séquences d'ADN codant lesdites autres protéines ou Epi A sont absentes.

5. Protéine caractérisée en ce qu'elle est codée par une molécule d'ADN selon l'une quelconque des revendications 1 à 4.

6. Procédé de production d'une protéine selon la revendication 5 caractérisé en ce qu'une molécule d'ADN telle que définie dans l'une quelconque des revendications 1 à 4 est insérée dans un vecteur plasmidique de sorte que ladite molécule d'ADN est sous le contrôle d'un promoteur-régulateur, l'insertion du vecteur plasmidique résultant dans un hôte approprié, la culture dudit hôte de sorte que la protéine est exprimée et l'isolement de la protéine.

7. Procédé de production d'une protéine ayant au moins l'une des caractéristiques structurales d'un antibiotique, caractérisé en ce qu'une séquence d'ADN codant une séquence d'acides aminés prédéterminée est insérée dans un plasmide vecteur de telle sorte qu'elle est sous le contrôle d'un promoteur-régulateur, la transformation au moyen du vecteur plasmidique résultant d'un hôte approprié et la culture de l'hôte de manière à exprimer ledit ADN et l'isolement de la protéine, où l'hôte est aussi transformé par une séquence d'ADN ou des séquences d'ADN telles que définies dans l'une quelconque des revendications 1 à 4 qui sont exprimées pendant ladite culture de l'hôte.

8. Procédé selon la revendication 7 caractérisé en ce que la séquence d'ADN codant la séquence d'acides aminés prédéterminée et la séquence d'ADN telle que définie dans l'une quelconque des revendications 1 à 3 sont insérées dans le même vecteur plasmidique que celui qui est utilisé pour transformer l'hôte.

9. Procédé selon la revendication 7 ou 8 caractérisé en ce que ladite séquence d'ADN contient une séquence d'ADN codant la séquence de prépeptide -30 à -1 de la pré-épidermine ou est insérée immédiatement en aval de celle-ci.

10. Procédé de production d'une protéine de fusion caractérisé en ce qu'une protéine selon la revendication 5 est fusionnée à une protéine auxiliaire.

11. Procédé de production d'une protéine de fusion caractérisé en ce qu'une protéine selon la revendication 5 est fusionnée à une protéine auxiliaire et une séquence clivable par voie enzymatique est introduite à la jonction entre la protéine selon la revendication 5 et la protéine auxiliaire.

12. Procédé selon la revendication 10 caractérisé en ce que ladite protéine auxiliaire facilite la sécrétion de la protéine de fusion à partir d'un hôte choisi.

13. Procédé selon la revendication 10 caractérisé en ce que la protéine auxiliaire facilite la purification par chromatographie d'affinité.

14. Procédé selon la revendication 10 caractérisé en ce que la protéine auxiliaire est la protéine de liaison au maltose provenant de E. coli.
